# EUROPEAN PATENT SPECIFICATION

(11) **EP 1 809 306 B1**
(45) Date of publication and mention of the grant of the patent: **12.09.2012**
(21) Application number: 05803225.1
(22) Date of filing: 07.11.2005
(51) Int. Cl.: A61K 33/34, A61P 17/02, A61P 17/00

(54) **COPPER CONTAINING MATERIALS FOR TREATING WOUNDS, BURNS AND OTHER SKIN CONDITIONS**
KUPFERHALTIGE MATERIALIEN ZUR BEHANDLUNG VON WUNDEN, VERBRENNUNGEN UND ANDEREN HAUTERKRANKUNGEN
MATERIAUX CONTENANT DU CUIVRE POUR LE TRAITEMENT DES BLESSURES, DES BRULURES ET D'AUTRES ETATS CUTANES

(30) Priority: 07.11.2004 IL 16506404; 07.11.2005 IL 17180705
(43) Date of publication of application: 25.07.2007
(73) Proprietor: The Cupron Corporation, Greensboro, NC 27410 (US)
(72) Inventor: GABBAY, Jeffrey, Jerusalem 92142 (IL)
(74) Representative: Fairbairn, Angus Chisholm
(86) International application number: PCT/IL2005/001160
(87) International publication number: WO 2006/048879

(56) References cited:
- WO-A-00/75415
- WO-A-01/74166
- WO-A-03/009877
- WO-A-03/018495
- WO-A-03/088983
- WO-A-2004/073756
- WO-A-2004/073758
- WO-A1-01/81617
- GB-A- 2 092 006

## Description

The present invention relates to a wound-healing material for use in treating sores, cold sores, cutaneous openings, ulcerations, abrasions, lesions and burns, incorporating water-insoluble copper compounds.

More particularly the present invention relates to wound-healing material for use as defined in claims 1-24 herein.

As will be described hereinafter with reference to the examples and the accompanying figures, it has now been surprisingly discovered that materials incorporating water-insoluble copper compounds which release Cu⁺ ions, Cu⁺⁺ ions or combinations thereof upon contact with a fluid can be used for the manufacture of a fabric, a film, a filament or a sheath to be brought into contact with a body surface having a wound such as a sore, a cold sore, a cutaneous opening, an ulceration, a lesion, including a vascular lesion and a mucosal lesion, an abrasion and a burn to effect the healing thereof.

More specifically, it has now been surprisingly found that the materials of the present invention are effective in healing ulcerative sores and/or lesions, such as those caused by diabetes, bed sores, burns, acne sores, herpes sores, and are also effective in the healing of sores associated with bacteria, fungus or virus such as eczema, psoriasis, herpes, etc.

In addition, the materials of the present invention can be used for treating nipple sores on nursing women and abrasion sores and lesions on the partial limbs of amputees. Pressure sores can also be treated with the materials of the present invention. Wounds and operational openings can be closed with suturing material made with the materials of the present invention and since the materials of the present invention have also been found to facilitate wound healing without scars as demonstrated e.g. in example 7 hereinafter, the suturing material of the present invention is especially useful in plastic surgery and other surgery in which esthetics are a factor.

In both WO 98/06508 and WO 98/06509 there are taught various aspects of a textile with a full or partial metal or metal oxide plating directly and securely bonded to the fibers thereof, wherein metal and metal oxides, including copper, are bonded to said fibers.

More specifically, in WO 98/06509 there is provided a process comprising the steps of: (a) providing a metallized textile, the metallized textile comprising: (i) a textile including fibers selected from the group consisting of natural fibers, synthetic cellulosic fibers, regenerated fibers, acrylic fibers, polyolefin fibers, polyurethane fibers, vinyl fibers, and blends thereof, and (ii) a plating including materials selected from the group consisting of metals and metal oxides, the metallized textile characterized in that the plating is bonded directly to the fibers; and (b) incorporating the metallized textile in an article of manufacture.

In the context of said invention the term "textile" included fibers, whether natural (for example, cotton, silk, wool, and linen) or synthetic yarns spun from those fibers, and woven, knit, and non-woven fabrics made of those yarns. The scope of said invention included all natural fibers; and all synthetic fibers used in textile applications, including but not limited to synthetic cellulosic fibers (i.e., regenerated cellulose fibers such as rayon, and cellulose derivative fibers such as acetate fibers), regenerated protein fibers, acrylic fibers, polyolefin fibers, polyurethane fibers, and vinyl fibers, but excluding nylon and polyester fibers, and blends thereof.

Said invention comprised application to the products of an adaptation of technology used in the electrolyses plating of plastics, particularly printed circuit boards made of plastic, with metals. See, for example, Encyclopedia of Polymer Science and Engineering (Jacqueline I. Kroschwitz, editor), Wiley and Sons, 1987, vol. IX, pp 580-598. As applied to textiles, this process included two steps. The first step was the activation of the textile by precipitating catalytic noble metal nucleation sites on the textile. This was done by first soaking the textile in a solution of a low-oxidation-state reductant cation, and then soaking the textile in a solution of noble metal cations, preferably a solution of Pd++ cations, most preferably an acidic PdCl₂ solution. The low-oxidation-state cation reduces the noble metal cations to the noble metals themselves, while being oxidized to a higher oxidation state. Preferably, the reductant cation is one that is soluble in both the initial low oxidation state and the final high oxidation state, for example Sn++, which is oxidized to Sn++++, or Ti+++, which is oxidized to Ti++++.

The second step was the reduction, in close proximity to the activated textile, of a metal cation whose reduction was catalyzed by a noble metal. The reducing agents used to reduce the cations typically were molecular species, for example, formaldehyde in the case of Cu++. Because the reducing agents were oxidized, the metal cations are termed "oxidant cations" herein. The metallized textiles thus produced were characterized in that their metal plating was bonded directly to the textile fibers.

In WO 98/06508 there is described and claimed a composition of matter comprising:
(a) a textile including fibers selected from the group consisting of natural fibers, synthetic cellulosic fibers, regenerated protein fibers, acrylic fibers, polyolefin fibers, polyurethane fibers, vinyl fibers, and blends thereof; and
(b) a plating including materials selected from the group consisting of metals and metal oxides;
the composition of matter characterized in that said plating is bonded directly to said fibers.

Said publication also claims a composition of matter comprising:
(a) a textile including fibers selected from the group consisting of natural fibers, synthetic cellulosic fibers, regenerated protein fibers, acrylic fibers, polyolefin fibers, polyurethane fibers, vinyl fibers, and blends thereof; and
(b) a plurality of nucleation sites, each of said nucleation sites including at least one noble metal;
the composition of matter characterized by catalyzing the reduction of at least one metallic cationic species to a reduced metal, thereby plating said fibers with said reduced metal.

In addition, said publication teaches and claims processes for producing said products.

A preferred process for preparing a metallized textile according to said publication comprises the steps of:
a) selecting a textile, in a form selected from the group consisting of yarn and fabric, said textile including fibers selected from the group consisting of natural fibers, synthetic cellulosic fibers, regenerated protein fibers, acrylic fibers, polyolefin fibers, polyurethane fibers, vinyl fibers, and blends thereof;
b) soaking said textile in a solution containing at least one reductant cationic species having at least two positive oxidation states, said at least one cationic species being in a lower of said at least two positive oxidation states;
c) soaking said textile in a solution containing at least one noble metal cationic species, thereby producing an activated textile; and
d) reducing at least one oxidant cationic species in a medium in contact with said activated textile, thereby producing a metallized textile.

Said publications, however, were limited to coated fibers and textiles prepared according to said processes for the uses described therein, however said publications did not teach or suggest that such coated fibers and textiles could be effective for treating and healing sores, cold sores, cutaneous openings, ulcerations, lesions, abrasions and burns as described and exemplified herein.

Similarly said publications did not teach or suggest the possibility of incorporating cationic copper into a polymeric slurry of a hydrophobic polymer whereby there are produced films and fibers having microscopic particles of cationic copper encapsulated therein and protruding there from which have now also been surprisingly discovered as being effective for treating and healing sores, cold sores, cutaneous openings, ulcerations, lesions, abrasions and burns as described and exemplified herein.

According to the description in US 2004/0224005 (WO 01/74166), it was discovered that by adding a small percentage of Cu++ in the form of water insoluble copper oxide particles to the slurry of a polymer to be formed, the resulting polymer possessed antimicrobial properties.

Furthermore it was surprisingly discovered and described therein that by adding copper oxide in particle form into a polymeric slurry of such polymers as polyethylene, polypropylene, polyesters and similar hydrophobic or hydrophilic polymeric materials it is possible to extrude fibers, yarns or sheets which possess both antimicrobial and antiviral properties which have a multiplicity of uses. Among the uses contemplated for the novel antimicrobial and antiviral polymeric materials described in said specification was their use in a backing for a carpet, which could even be used in a hospital setting since it would not develop mold, smell, and would inactivate any viruses settling thereon; the use as a component of a molded non-woven product such as an air filter in a hospital or airplane or a mask which could be made air permeable or liquid permeable and be used to filter fluids flowing there through and to inactivate bacteria and viruses found in said fluids; formation into a continuous, flat, textured or stretched form which could be used in articles of clothing such as stockings, socks, shirts or any article of clothing that would incorporate a hydrophobic polymeric fiber or yarn; formation of a short staple fiber which could be then used as is or blended with other fibers such as cotton, which blended yarns could then be used for the manufacture of a variety of both knit and woven products such as socks, sheets, etc.; and use of such polymeric materials, manufactured in the form of a bi-component yarn in which the core is one compound and the sheath around the core is a polymer containing the water insoluble copper oxide particles creating a yarn with a multitude of end uses in either a continuous, flat, textured, stretched form or as a short staple. An example of said latter use would be the use of a polyethylene core with a polymeric sheath incorporating said water insoluble copper oxide particles to form a yarn with an increased resistance to being cut or ripped while also being both antimicrobial and antiviral and having a multiplicity of uses including in the food preparation industry.

Said material was described as being made from almost any synthetic polymer, which will allow the introduction of an cationic, copper oxide particles into its liquid slurry state. Examples of some materials are polyamides (nylon), polyester, acrylic, and polyalkylenes such as polyethylene and polypropylene, When the copper oxide dust is ground down to fine powder, e.g., a size of between 1 and 10 microns and introduced into the slurry in small quantities, e. g., in an amount of between 0.25 and 10% of the polymer weight, in a master batch as is the accepted practice for manufacturing extruded fibers and films it was found that the subsequent product produced from this slurry exhibited both antimicrobial and antiviral properties.

Unlike the fibers described, e. g. in WO 98/06508 and WO 98/06509, in which the fibers are coated on the outside, in said product the polymer has microscopic water insoluble particles of cationic copper oxide encapsulated therein with a portion of said particles being exposed and protruding from surfaces thereof. These exposed particles which protrude from the surface of the polymeric material have been shown to be active, as demonstrated by the tests set forth in said specification.

Said US specification, however, also did not teach or suggest that the polymeric materials described therein are effective for treating and healing sores, cold sores, cutaneous openings, ulcerations, lesions, abrasions and burns as described and exemplified herein.

In general, the products of said specification and also products which can be used in the present invention are produced as follows:
1. A slurry is prepared from any polymer, the chief raw material preferably being selected from a polyamide, a polyalkylene, a polyurethane and a polyester. Combinations of more than one of said materials can also be used provided they are compatible or adjusted for compatibility. The polymeric raw materials are usually in bead form and can be mono-component, bi-component or multi-component in nature. The beads are heated to melting at a temperature which preferably will range from about 120 to 180 °C.
2. At the hot mixing stage, before extrusion, a water insoluble powder of cationic copper oxide is added to the slurry and allowed to spread through the heated slurry The particulate size will be preferably between 1 and 10 microns, however can be larger when the film or fiber thickness can accommodate larger particles.
3. The liquid slurry is then pushed with pressure through holes in a series of metal plates formed into a circle or other desired shape called a spinneret. As the slurry is pushed through the fine holes that are close together, they form single fibers or if allowed to contact one another, they form a film or sheath. The hot liquid fiber or film is pushed upward with cold air forming a continuous series of fibers or a circular sheet. The thickness of the fibers or sheet is controlled by the size of the holes and speed at which the slurry is pushed through the holes and upward by the cooling air flow.

In WO 94/15463 there are described antimicrobial compositions comprising an inorganic particle with a first coating providing antimicrobial properties and a second coating providing a protective function wherein said first coating can be silver or copper or compounds of silver, copper and zinc and preferred are compounds containing silver and copper (II) oxide. Said patent, however, is based on the complicated and expensive process involving the coating of the metallic compositions with a secondary protective coating selected from silica, silicates, borosilicates, aluminosilicates, alumina, aluminum phosphate, or mixtures thereof and in fact all the claims are directed to compositions having successive coatings including silica, hydrous alumina and dioctyl azelate.

In contradistinction, the present invention is inter alia directed to the use of a polymeric material, having microscopic water insoluble particles of cationic copper oxide in powder form, which release Cu⁺⁺ encapsulated therein with a portion of said particles being exposed and protruding from surfaces thereof, which is neither taught nor suggested by said publication and which has the advantage that the exposed Cu⁺⁺ releasing water insoluble particles which protrude from the polymeric material have been proven to be effective in open wound healing.

In EP 427858 there is described an antibacterial composition characterized in that inorganic fine particles are coated with an antibacterial metal and/or antibacterial metal compound and said patent does not teach or suggest a polymer that incorporates microscopic water insoluble particles of cationic copper oxide in powder form, which release Cu⁺⁺ encapsulated therein with a portion of said particles being exposed and protruding from surfaces thereof.

In DE 4403016 there is described a bactericidal and fungicidal composition utilizing copper as opposed to ionic Cu⁺⁺ and said patent also does not teach or suggest a polymer that incorporates microscopic water insoluble particles of cationic copper oxide in powder form, which release Cu⁺⁺ encapsulated therein with a portion of said particles being exposed and protruding from surfaces thereof.

In JP-01 046465 there is described a condom releasing sterilizing ions utilizing metals selected from copper, silver, mercury and their alloys which metals have a sterilizing and sperm killing effect, wherein the metal is preferably finely powdered copper. While copper salts such as copper chloride, copper sulfate and copper nitrate are also mentioned, as is known, these are water soluble salts which will dissolve and break down the polymer in which they are introduced. Similarly, while cuprous oxide is specifically mentioned, this is a Cu⁺ ionic form, and therefore said patent does not teach or suggest the use of exposed Cu⁺⁺ releasing water insoluble particles which protrude from the polymeric material and which have been proven to be effective in open wound healing.

In JP-01 246204 there is described an antimicrobial molded article in which a mixture of a powdery copper compound and organic polysiloxane are dispersed into a thermoplastic molded article for the preparation of cloth, socks, etc. Said patent specifically states and teaches that metal ions cannot be introduced by themselves into a polymer molecule and requires the inclusion of organopolysiloxane which is also intended to provide a connecting path for the release of copper ions to the fiber surface. Thus, as will be realized said copper compound will be encapsulated and said patent does not teach or suggest the use of exposed Cu⁺⁺ releasing water insoluble copper oxide particles that protrude from the polymeric material.

In JP-03 113011 there is described a fiber having good antifungal and hygienic action preferably for producing underwear wherein said synthetic fiber contains copper or a copper compound in combination with germanium or a compound thereof, however, said patent teaches and requires the presence of a major portion of germanium and the copper compounds disclose therein are preferably metallic copper, cuprous iodide which is a monovalent Cu⁺ compound and water soluble copper salts. Thus, said patent does not teach or suggest the use of exposed Cu⁺⁺ releasing water insoluble copper oxide particles wh ich protrude from the polymeric material.

In EP 116865 there is described and claimed a polymer article containing zeolite particles at least part of which retain at least one metal ion having a bacterial property and thus said patent does not teach or suggest the use of exposed Cu⁺⁺ releasing water insoluble copper oxide particles, by themselves and in the absence of a zeolite, which particles protrude from the polymeric material and which have been proven to be effective in open wound healing.

In EP 253653 there is described and claimed a polymer containing amorphous aluminosilicate particles comprising an organic polymer and amorphous aluminosilicate solid particles or amorphous aluminosilicate solid particles treated with a coating agent, at least some of said amorphous aluminosilicate solid particles holding metal ions having a bactericidal actions. Thus, said patent does not teach or suggest the use of exposed Cu⁺⁺ releasing water insoluble copper oxide particles, by themselves and in the absence of amorphous aluminosilicate particles, which exposed Cu⁺⁺ releasing water insoluble copper oxide particles, protrude from the polymeric material and which have been proven to be effective in open wound healing.

In GB-A-2 092 006 is described a copper-coated ganze for use as a germicide (bacteriocide) in a wound dressing.

Thus none of said publications teach or suggest the use of water-insoluble copper compounds which release Cu⁺ ions, Cu⁺⁺ ions or combinations thereof upon contact with a fluid for the manufacture of a fabric or an extruded film, filament or sheath to be brought in contact with a body surface having sores, abrasions, ulcerations, lesions, cutaneous openings and burns for the treatment and healing thereof.

Thus, one preferred aspect of the present invention relates to the use of fibers incorporating water-insoluble copper compounds which release Cu⁺ ions, Cu⁺⁺ ions or combinations thereof upon contact with a fluid for the manufacture of a bandage for the treatment of sores, cold sores, cutaneous openings, ulcerations, lesions, abrasions and burns.

In preferred embodiments of said aspect of the invention, said bandage is formed of a gauze material having said copper compounds incorporated therein.

A second preferred aspect of the present invention relates to the use of a polymeric film having microscopic water insoluble particles of ionic copper oxides in powdered form, embedded directly therein with a portion of said particles being exposed and protruding from surfaces thereof, which particles release Cu⁺ ions, Cu⁺⁺ ions or combinations thereof upon contact with a fluid for the manufacture of a bandage for the treatment of sores, cold sores, cutaneous openings, ulcerations, lesions, abrasions and burns.

In preferred embodiments of said second aspect of the present invention, said fibers are polymeric fibers having said compounds incorporated therein and protruding from the surfaces thereof.

In other preferred embodiments of said aspect of the present invention said fibers are coated with said copper compounds

A third preferred aspect of the present invention relates to the use of fibers incorporating water-insoluble copper compounds which release Cu⁺ ions, Cu⁺⁺ ions or combinations thereof upon contact with a fluid for the manufacture of patient attire for hospital and health care facilities, such as nursing homes, senior citizen residences, chronic care facilities, rehabilitation centers, and hospices to prevent the formation of bed sores and to treat such sores if formed.

As is known, in patients such as invalids and chronically ill and elderly patients who are confined to a bed or a wheel chair for extensive periods of time, pressure sores and bed sores often lead to life-threatening complications.

Thus according to the present invention, by providing a garment such as pajamas, nightgowns and underwear incorporating water-insoluble copper compounds which release Cu⁺ ions, Cu⁺⁺ ions or combinations thereof upon contact with a fluid, at least in the area of the garment which lies adjacent to the buttock area of the patient, one can prevent or immediately effect healing of such sores at their inception.

More specifically this aspect of the present invention also relates to the use of water-insoluble copper compounds which release Cu⁺ ions, Cu⁺⁺ ions or combinations thereof upon contact with a fluid for the manufacture of a garment selected from the group consisting of pajamas, nightgowns and underwear for patient attire for hospital and health care facilities, said garment having a panel including water-insoluble copper compounds which release Cu⁺ ions, Cu⁺⁺ ions or combinations thereof upon contact with a fluid incorporated at least in the area of the garment which lies adjacent to the buttocks area of a patient for the prevention and healing of bed and pressure sores.

As will be realized materials of the present invention can also be incorporated in other areas of garments to be positioned adjacent to other areas of the body which are prone to suffer from the formation of pressure sores.

Also in this third aspect of the present invention said fibers are preferably polymeric fibers having said compounds incorporated therein and protruding from the surfaces thereof or said fibers are coated with said copper compounds

A fourth preferred aspect of the present invention relates to the use of a polymeric film having microscopic water insoluble particles of ionic copper oxides in powdered form, embedded directly therein with e portion of said particles being exposed and protruding from surfaces thereof, which particles release Cu⁺ ions, Cu⁺⁺ ions or combinations thereof upon contact with a fluid for the manufacture of a protective sheath for a body limb for the treatment of sores forming thereon.

A fifth preferred aspect of the present invention relates to the use of fibers incorporating water-insoluble copper compounds which release Cu⁺ ions, Cu⁺⁺ ions or combinations thereof upon contact with a fluids for the manufacture of a protective sheath for a body limb for the treatment of sores forming thereon.

Also in this fifth aspect of the present invention said fibers are preferably polymeric fibers having said compounds incorporated therein and protruding from the surfaces thereof or said fibers are coated with said copper compounds.

A sixth preferred aspect of the present invention relates to the use of fibers incorporating water-insoluble copper compounds which release Cu⁺ ions, Cu⁺⁺ ions or combinations thereof upon contact with a fluid for the manufacture of a dressing for the treatment of sores, cold sores, cutaneous openings, ulcerations, lesions, abrasions and burns.

Also in this sixth aspect of the present invention said fibers are preferably polymeric fibers having said compounds incorporated therein and protruding from the surfaces thereof or said fibers are coated with said copper compounds.

A seventh preferred aspect of the present invention relates to the use of a polymeric film having microscopic water insoluble particles of ionic copper oxides in powdered form, embedded directly therein with a portion of said particles being exposed and protruding from surfaces thereof, which particles release Cu⁺ ions, Cu⁺⁺ ions or combinations thereof upon contact with a fluid for the manufacture of a dressing for the treatment of sores, cold sores, cutaneous openings, ulcerations, lesions, abrasions and burns.

An eighth preferred aspect of the present invention relates to the use of fibers incorporating water-insoluble copper compounds which release Cu⁺ ions, Cu⁺⁺ ions or combinations thereof upon contact with a fluid for the manufacture of underpants for men for treating the outbreak of male genital herpes sores.

Also in this eighth aspect of the present invention said fibers are preferably polymeric fibers having said compounds incorporated therein and protruding from the surfaces thereof or said fibers are coated with said copper compounds.

A ninth preferred aspect of the present invention relates to the use of fibers incorporating water-insoluble copper compounds which release Cu⁺ ions, Cu⁺⁺ ions or combinations thereof upon contact with a fluid for the manufacture of bras and nursing pads for nursing mothers for the treatment of nipple sores.

Also in this ninth aspect of the present invention said fibers are preferably polymeric fibers having said compounds incorporated therein and protruding from the surfaces thereof or said fibers are coated with said copper compounds.

A tenth preferred aspect of the present invention relates to the use of fibers incorporating water-insoluble copper compounds which release Cu⁺ ions, Cu⁺⁺ ions or combinations thereof upon contact with a fluid for the manufacture of a dressing for the treatment of acne sores.

Also in this tenth aspect of the present invention said fibers are preferably polymeric fibers having said compounds incorporated therein and protruding from the surfaces thereof or said fibers are coated with said copper compounds.

In especially preferred embodiment of this tenth aspect of the present invention, said fibers are incorporated into the pad of a padded adhesive bandage.

An eleventh preferred aspect of the present invention relates to the use of fibers incorporating water-insoluble copper compounds which release Cu⁺ ions, Cu⁺⁺ ions or combinations thereof upon contact with a fluid for the manufacture of a fabric to be brought in contact with a body surface affected by psoriasis for the treatment thereof.

Also in this eleventh aspect of the present invention said fibers are preferably polymeric fibers having said compounds incorporated therein and protruding from the surfaces thereof or said fibers are coated with said copper compounds.

A twelfth preferred aspect of the present invention relates to the use of fibers incorporating water-insoluble copper compounds which release Cu⁺ ions, Cu⁺⁺ ions or combinations thereof upon contact with a fluid for the manufacture of a fabric to be brought in contact with a body surface affected by eczema for the treatment thereof.

Also in this twelfth aspect of the present invention said fibers are preferably polymeric fibers having said compounds incorporated therein and protruding from the surfaces thereof or said fibers are coated with said copper compounds.

A thirteenth preferred aspect of the present invention relates to the use of fibers incorporating water-insoluble copper compounds which release Cu⁺ ions, Cu⁺⁺ ions or combinations thereof upon contact with a fluid, for the manufacture of a fabric to be brought in contact with a body surface having sores, abrasions and burns for the treatment and healing thereof.

A fourteenth preferred aspect of the present invention relates to the use of fibers incorporating water-insoluble copper compounds which release Cu⁺ ions, Cu⁺⁺ ions or combinations thereof upon contact with a fluid, for the manufacture of a suturing material.

A fifteenth preferred aspect of the present invention relates to the use of polymeric filament having microscopic water insoluble particles of ionic copper oxides in powdered form, embedded directly therein with a portion of said particles being exposed and protruding from surfaces thereof, which particles release Cu⁺ ions, Cu⁺⁺ ions or combinations thereof upon contact with a fluid for the manufacture of a suturing material.

A sixteenth preferred aspect of the present invention relates to the use of water-insoluble copper compounds which release Cu⁺ ions, Cu⁺⁺ ions or combinations thereof upon contact with a fluid for the manufacture of a wound-healing fabric to be used in a military uniform or article of inner or outer clothing, said fabric including fibers which upon entry into a wound of a military personnel sustaining a wound while wearing the same, achieves both an anti-bacterial effect and a healing effect on said wound.

A preferred embodiment of this aspect of the invention relates to the use of fibers incorporating water-insoluble copper compounds which release Cu⁺ ions, Cu⁺⁺ ions or combinations thereof upon contact with a fluid for the manufacture of a military uniform or article of inner or outer clothing, which fibers upon entry into a wound of a military personnel sustaining a wound while wearing the same, achieves both an anti-bacterial effect and a healing effect on said wound.

Also in this aspect of the present invention said fibers are preferably polymeric fibers having said compounds incorporated therein and protruding from the surfaces thereof or said fibers are coated with said copper compounds.

As is known a problem that has existed for the military is that when military personnel are wounded, e.g. by projectiles, shrapnel, and explosions, often fibers from the uniforms or articles of inner or outer clothing that they are wearing are driven into the wounds that are created and can themselves be a source of infection. Thus, the above aspect of the present invention addresses this problem by providing military personnel with uniforms and articles of inner or outer clothing that, in the unfortunate event that the wearer thereof is wounded, not only will exert an anti-bacterial effect to prevent infection, but will also actively enhance the healing of the sustained wound.

A yet further use of the materials of the present invention is in the formation of the inner lining of a cast_{.}

The present invention may be useful in a method for treating sores, cold sores, cutaneous openings, ulcerations, lesions, abrasions and burns comprising applying thereto a polymeric material formed from a polymeric component selected from the group consisting of a polyamide, a polyester, an acrylic and a polyalkylene, said material being in the form of a fiber, a yam, a sheath, a filament, or a sheet, and having microscopic water insoluble particles of ionic copper oxides in powdered form, embedded directly therein with a portion of said particles being exposed and protruding from surfaces thereof, which particles release Cu⁺ ions, Cu⁺⁺ ions or combinations thereof upon contact with a fluid.

In a further preferred embodiment, the present invention may be useful in a method for preventing the formation of diabetic granulation, lesions and ulcers comprising applying, a material incorporating water-insoluble copper compounds which release Cu⁺ ions, Cu⁺⁺ ions or combinations thereof upon contact with a fluid, to an area to be protected.

The present invention may be useful in a method for preventing the formation of diabetic granulation, lesion s and ulcers comprising applying a polymeric material formed from a polymeric component selected from the group consisting of a polyamide, a polyester, an acrylic and a polyalkylene, said material being in the form of a fiber, a yarn, a sheath, a filament, or a sheet, and having microscopic water insoluble particles of ionic copper oxides in powdered form, embedded directly therein with a portion of said particles being exposed and protruding from surfaces thereof, which particles release Cu⁺ ions, Cu⁺⁺ ions or combinations thereof upon contact with a fluid, to an area to be protected.

In especially preferred embodiments of this aspect of the invention, said material is a fabric having fibers incorporating water-insoluble copper compounds which release Cu⁺ ions, Cu⁺⁺ ions or combinations thereof upon contact with a fluid.

As stated, the present invention relates to a use for treating and healing sores, cold sores, cutaneous openings, ulcerations, lesions, abrasions and burns comprising applying to a body surface exhibiting the same, a material incorporating water-insoluble copper compounds which release Cu⁺ ions, Cu⁺⁺ ions or combinations thereof upon contact with a fluid to effect the treatment and healing thereof.

In a first preferred embodiment said sore is an ulcerative sore.

In a second preferred embodiment said sore is a bed sore.

In an especially preferred embodiment said sore is an ulcerative sore caused by diabetes.

In yet another preferred embodiment said lesion is a vascular lesion.

In a further preferred embodiment said lesion is a mucosal lesion.

In a first group of preferred embodiments of the present invention said material is a fabric having fibers incorporating water-insoluble copper compounds which release Cu⁺ ions, Cu⁺⁺ ions or combinations thereof upon contact with a fluid.

In a second group of preferred embodiments said material is a polymeric film having microscopic water insoluble particles of ionic copper oxides in powdered form, embedded directly therein with a portion of said particles being exposed and protruding from surfaces thereof, which particles release Cu⁺ ions, Cu⁺⁺ ions or combinations thereof upon contact with a fluid.

In a third group of preferred embodiments of the present invention said material is a polymeric fiber having microscopic water insoluble particles of ionic copper oxides in powdered form, embedded directly therein with a portion of said particles being exposed and protruding from surfaces thereof, which particles release Cu⁺ ions, Cu⁺⁺ ions or combinations thereof upon contact with a fluid.

In a fourth group of preferred embodiments of the present invention said material is a polymeric filament having microscopic water insoluble particles of ionic copper oxides in powdered form, embedded directly therein with a portion of said particles being exposed and protruding from surfaces thereof, which particles release Cu⁺ ions, Cu⁺⁺ ions or combinations thereof upon contact with a fluid.

In a fifth group of preferred embodiments of the present invention said material is a polymeric sheath having microscopic water insoluble particles of ionic copper oxides in powdered form, embedded directly therein with a portion of said particles being exposed and protruding from surfaces thereof, which particles release Cu⁺ ions, Cu⁺⁺ ions or combinations thereof upon contact with a fluid.

In a sixth group of preferred embodiments of the present invention, said material is a polymeric film having microscopic water insoluble particles of ionic copper oxides in powdered form, embedded directly therein with a portion of said particles being exposed and protruding from surfaces thereof, which particles release Cu+ ions, Cu++ ions or combinations thereof upon contact with a fluid wherein said film has the ability to disperse liquid through osmosis.

In a seventh group of preferred embodiments of the present invention, said material is a polymeric film having microscopic water insoluble particles of ionic copper oxides in powdered form, embedded directly therein with a portion of said particles being exposed and protruding from surfaces thereof, which particles release Cu+ ions, Cu++ ions or a combination thereof upon contact with a fluid wherein said film has micro pores perforated throughout to allow for the escape of excess liquids.

As stated above, based on the surprising discovery of the present invention that a material incorporating water-insoluble copper compounds which release Cu⁺ ions, Cu⁺⁺ ions or combinations thereof, upon contact with a fluid, can be used in a method for treating and healing sores, cold sores, cutaneous openings, ulcerations, lesions, abrasions and burns to effect the treatment and healing of affected surfaces by applying said material to such an affected body surface, it has now been realized that the present method is effective for treating many conditions.

Thus, the present invention may be useful in a method for treating an outbreak of male genital herpes sores comprising providing underpants having fibers incorporating water-insoluble copper compounds which release Cu⁺ ions, Cu⁺⁺ ions or combinations thereof upon contact with a fluid.

The present invention also may be useful in a method for treating acne sores comprising applying thereto a fabric having fibers incorporating water-insoluble copper compounds which release Cu⁺ ions, Cu⁺⁺ ions or combinations thereof upon contact with a fluid.

Similarly, the present invention may be useful in a method for treating nipple sores on nursing women comprising providing a bra or nursing pad having fibers incorporating water-insoluble copper compounds which release Cu⁺ ions, Cu⁺⁺ ions or combinations thereof upon contact with a fluid.

In addition, the present invention may be useful in a method for treating burns, comprising providing a wound-healing fabric or an extruded wound-healing film, or filament incorporating water-insoluble copper compounds which release Cu⁺ ions, Cu⁺⁺ ions or combinations thereof upon contact with a fluid for application to said burn surface.

The polymeric materials for use in the present invention can be produced by preparing a slurry of a polymer selected from the group consisting of a polyamide, a polyester, an acrylic and a polyalkylene, and mixtures thereof, introducing a powder consisting essentially of water insoluble cationic copper oxides and dispersing the same in said slurry and then extruding said slurry to form a polymeric material wherein water insoluble copper oxide particles that release Cu⁺⁺ are encapsulated therein with a portion of said particles being exposed and protruding from surfaces thereof, which polymeric material is then formed into a fiber, a yarn or a sheet to be manufactured into a fabric suitable to be brought in contact with a body surface having sores, abrasions and burns for the treatment and healing thereof.

In US Patent 6,124,221 (WO 00/75415) there is described and claimed an article of clothing having antibacterial, antifungal, and antiyeast properties, comprising at least a panel of a metallized textile, the textile including fibers selected from the group consisting of natural fibers, synthetic cellulosic fibers, regenerated protein fibers, acrylic fibers, polyolefin fibers, polyurethane fibers, vinyl fibers, and blends thereof, and having a plating including an antibacterial, antifungal and antiyeast effective amount of at least one oxidant cationic species of copper.

In said specification there was described that said article of clothing was effective against *Tinea Pedis,* against *Candida Albicans,* against *Thrush* and against bacteria causing foot odor, selected from the group of *brevubacterium, acinetobacter,* micrococcus and combinations thereof, however said patent did not teach or suggest that such an article of clothing were intended for use or would be effective in the treatment of wounds such as sores, cold sores, cutaneous openings, ulcerations, lesions, abrasions and burns.

In WO 01/81671 there is described that textile fabrics incorporating fibers coated with a cationic form of copper are also effective for the inactivation of antibiotic resistant strains of bacteria and said cationic species of copper preferably comprises Cu⁺⁺ ions, however, also in this specification, the textile fabrics were described for use in treating a hospital environment to prevent the spread of infection by the inactivation of such bacteria excreted by an infected patient and said specification did not teach or suggest that an article of clothing formed from such a textile fabric would be effective in the treatment of wounds such as sores, cold sores, cutaneous openings, ulcerations, lesions, abrasions and burns.

In WO 01/74166 there is described and claimed the use of particles which release Cu⁺⁺ for the preparation of a polymeric material having microscopic particles which release Cu⁺⁺ encapsulated therein with a portion of said particles being exposed and protruding from surfaces thereof, said polymeric material being effective to inhibit HIV-1 proliferation, however, said publication was limited to the teaching of the use of such polymeric materials for the preparation of condoms and possibly gloves and the inventor thereof did not realize at said time and said publication does not teach or suggest the present inventive concept of providing an article of clothing which would be effective in the treatment of wounds such as sores, cold sores, cutaneous openings, ulcerations, lesions, abrasions and burns.

In US Patent 5848592, US Patent 5492882 , French patent 2764518, British Patent 1382820 and US Patent 5217626 there are variously disclosed air or water filters comprising copper metal, copper oxides, chloride, carbonate and sulfate against noxious vapors and gases and against bacteria and viruses. In the case of British Patent 1382820 a gas filter is disclosed incorporating active carbon and/or an oxide or oxides of one or more metals of a high molecular weight in order to physically block and prevent the passage of bacteria. In the case of US patent 5215626 a water filter is disclosed incorporating a mixture of a permanganate compound, a silver compound and a water-soluble copper compound such as copper chloride or copper sulfate.

None of said references however, teach or suggest the use of fibers incorporating water-insoluble copper compounds which release Cu⁺ ions, Cu⁺⁺ ions or combinations thereof upon contact with a fluid, for the manufacture of a fabric to be brought in contact with a body surface having sores, cold sores, cutaneous openings, ulcerations, abrasions, lesions and burns for the treatment and healing thereof.

DATABASE WPI Section Ch, Week 199031 Derwent Publications Ltd,. London, GB; Class BO4, An 1990-234808 XP002247181 & JP 02 161954 and DATABASE WPI Section Ch, Week 198821 Derwent Publications Ltd,. London, GB; Class A88, An 1988-145060 XP002247182 & JP 63 1088007 relate to hollow porous fibres and especially JP 631088007 discloses treating body fluids with cellulose bound copper ammonium however neither of said references teach or suggest the use of fibers incorporating water-insoluble copper compounds which release Cu⁺ ions, Cu⁺⁺ ions or combinations thereof upon contact with a fluid, for the manufacture of a fabric to be brought in contact with a body surface having sores, cold sores, cutaneous openings, ulcerations, abrasions, lesions and burns for the treatment and healing thereof.

As stated hereinbefore WO 01/74166 teaches and claims an antimicrobial and antiviral polymeric material, having microscopic particles which release Cu⁺⁺ encapsulated therein and protruding from surfaces thereof but does not teach or suggest the method of the present invention. Similarly WO 01/81671 teaches and claims a method for combating and preventing nosocomial infections, comprising providing to health care facilities textile fabrics incorporating fibers coated with a cationic form of copper, for use in patient contact and care, wherein said textile fabric is effective for the inactivation of antibiotic resistant strains of bacteria and also does not teach or suggest the use of fibers incorporating water-insoluble copper compounds which release Cu⁺ ions, Cu⁺⁺ ions or combinations thereof upon contact with a fluid, for the manufacture of a fabric to be brought in contact with a body surface having sores, cold sores, cutaneous openings, ulcerations, abrasions, lesions and burns for the treatment and healing thereof.

Thus, none of the above publications teach or suggest the subject matter of the present invention.

In the use of the present invention the cationic species of copper must be exposed to a liquid medium to allow for atomic dispersion into the medium whether said medium is body fluid from an open wound, sore or burn, perspiration which acts as a carrier for said atomic dispersion, or a liquid or surfactant which is added to the fibers or fabric to facilitate the transfer of the ions to the site of the sore, abrasion or bum.

In order to form a wound-treating material of the present invention one would preferably take fibers having ionic copper selected from the group consisting of Cu⁺ and Cu⁺⁺ ions and include them in a substrate. In a woven substrate, the fibers would be blended with any other fiber and woven or knit into a substrate. In a non-woven configuration the fibers would be blended to form a thin layer. In both cases, a number of layers could preferably be placed one on top of the other to form a pad.

The ionic copper used in the present invention is prepared in a manner similar to that described in the earlier specifications referenced above with slight modifications as described hereinafter and is obtained through a redox reaction either on a substrate or alone in the liquid. The method of production is an adaptation of technology as used in the electroless plating of plastics, particularly printed circuit boards made of plastic, with metals. See, for example, Encyclopedia of Polymer Science and Engineering (Jacqueline I. Kroschwitz, editor), Wiley and Sons, 1987, vol. IX, pp 580-598. As applied to fibers or fabrics or membranes, this process includes two steps. The first step is the activation of the substrate by precipitating a catalytic noble metal nucleation sites on the substrate surface. This is done by first soaking the substrate in a solution of a low-oxidation-state reductant cation, and then soaking the substrate in a solution of noble metals cations, preferably a solution of Pd++ cations, most preferable an acidic PdCl₂ solution. The low-oxidation-state cation reduces the noble metal cations to the noble metals themselves, while being oxidized to a higher oxidation state. Preferable, the reductant cation is one that is soluble in both the initial low oxidation state and the final high oxidation state, for example Sn++, which is oxidized to Sn++++, or Ti+++. which is oxidized to Ti++++.

The second step is the reduction, in close proximity to the activated substrate, of a metal cation whose reduction is catalyzed by a noble metal. The reducing agents used to reduce the cations typically are molecular species, for example, formaldehyde in the case of Cu++. Because the reducing agents are oxidized, the metal cations are termed "oxidant cations" herein. The metallized substrate thus produced is characterized in that their metal plating is bonded directly to the substrate.

Based on the process described above, it is also possible for someone familiar with the art to identify the oxidant states by their colors. When the substrate is allowed to float in a copper solution for reduction as described above, different colors are obtained on each side of the substrate. The topside of the substrate is the shiny bright copper (red/yellow) color characteristic of elemental copper - Cu. The bottom side of the fabric is a black color, which is characteristic of CuO. Any substrate located under the top substrate also shows a black shade on its upper side.

In the process described herein, changes are made to the process to allow the plating of a cellulose fiber or substrate with a different cation ic species of copper than elemental copper or copper oxide (CuO - black).

This form of electro-less plating process involves the reduction of a cationic form of copper from a copper solution such as copper sulfate or copper nitrate on to a prepared surface on fibers or a substrate. The fibers or substrate to be plated must first be soaked in a solution containing at least one reductant cationic species having at least two positive oxidation states, then at least one cationic species being in a lower of the at least two positive oxidation states. The fibers or substrate are then soaked in a solution containing at least one noble metal cationic species, thereby producing an activated surface.

The fibers are then exposed to at least one oxidant cationic species in a medium in contact with the activated surface. A reducing agent is then added and the copper reduces itself from the solution on to the surface of the fibers. Without the following changes, the fibers or substrate produced using this formula demonstrates an elemental copper coating on the fibers which are on the top of the fiber or substrate pack and black colored fibers below and throughout the fiber or substrate pack.

As stated hereinbefore, in order to obtain a surface that is effective for the treatment of sores, cold sores, cutaneous openings, ulcerations, lesions, abrasions , burns and skin conditions a cationic species of copper must be obtained. The effective compounds of copper must contain either a Cu (I) or Cu (II) species or both. To insure obtaining these species on cellulose, the Pd++ must be applied so that there is equal saturation of all fibers at the same time, e.g. by soaking and squeezing. If a large fiber pack is dropped into the Pd++ solution, the first fibers to hit the solution will absorb more of the Pd++ solution than other parts of the pack, which will upset the cationic copper deposition. In addition, the fibers must be washed between the first process involving the Sn++ and the second process, Pd++, in water. Residual Sn++ solution left between the fibers will cause a reduction of the Pd++ directly into the solution between the fibers and will allow only a random reduction of the Pd++ on the fibers which will again effect the deposition of the copper. While these two points may seem small, they have a direct effect on the plating.

In addition, a change is necessary in the application system of the copper solution to the process. A side effect of the reduction process on to the fibers is the creation of hydrogen. This hydrogen appears as bubbles on the surface of the fibers. The hydrogen forms as a result of the interaction in the copper solution with the Pd++ on the fiber surface. If the hydrogen is not removed, by methods known per se, such as squeezing, from the surface of the fibers immediately upon their formation, the fibers exposed to the air will be coated with an elemental copper. The fibers just below the surface of the elemental copper will be black copper oxide. If, however, the hydrogen is removed immediately with their formation of the bubbles, the desired cationic species is obtained throughout the fiber pack. The desired color will be a dark brown which is distinct from the copper metal color or the black copper oxide. A further indication of the cationic species is that the fibers will not conduct electricity.

This process yields both a Cu (I) and a Cu (II) species as part of copper oxide compounds. Analysis of residual copper oxide powder formed by this process has shown that formed on the surface are copper oxide compounds which are 70% Cu (I), and 30% Cu (II). These compounds have been proven to be a highly effective in the treatment of sores abrasions, burns and skin conditions. The activity of the copper takes advantage of the redox reaction of the cationic species with water and allows a switch between Cu(II) and Cu (I) when there is contact with water. Cu(I) is more effective than Cu(II) against HIV while Cu(II) is more stable than Cu(I).

In US 2003/0198945 (WO 03/086478), there is described and claimed a device for the inactivation of a virus comprising a filtering material, said device having ionic copper selected from the group consisting of Cu⁺ and Cu⁺⁺ ions and combinations thereof incorporated therein.

In said specification there is described the plating of cellulose fibers using a copper solution which results in the formation of copper oxide on the surface of said fibers wherein the process used yields both a Cu(I) and a Cu(II) species as part of a copper oxide molecule. Said fibers were then incorporated into a filter which was found to be effective in the inactivation of HIV-1. Further tests with said filter revealed that this combination was also effective in the inactivation of West Nile fever virus and the neutralization of adenovirus and therefore it is believed that the antiviral hydrophilic polymeric materials of the present invention are also effective against such viruses since they work on the same mechanism.

While the mechanism of the hydrophilic polymeric materials used according to the present invention is not fully understood, in light of the results obtained, it is believed that when the polymeric material is brought into contact with a fluid aqueous medium, said medium leaches the cationic species of copper from within said polymer and as described in WO 03/086478 the antiviral activity takes advantage of the redox reaction of the cationic species with water and allows a switch between Cu (II) and Cu (I) when there is contact with water. Cu(I) is more effective than Cu(II) while Cu(II) is more stable than Cu(I). The Cu(II) compound will oxidize much more slowly than the Cu(I) compound and will increase the shelf life of the product.

As stated hereinbefore, the discovery of the present invention that materials incorporating water-insoluble copper compounds which release Cu⁺ ions, Cu⁺⁺ ions or combinations thereof upon contact with a fluid are surprisingly effective in healing wounds and even healing open wounds including the astounding discovery that such material can bring about the healing of ulcerative sores caused by diabetes, vascular lesions and similar wounds which heretofore were considered to be difficult, if not impossible to heal, enables the production of an entire new line of products according to the present invention.

Thus the following is a description of some products and the protocols for their use:
Wound dressings: This can be in the form of a woven gauze or solid thin film. When in the form of gauze, the material is placed over the wound and taped or held in place as is common practice for the use of a non-treated sterile gauze. If a film is used, than the film is placed over the wound area and taped down around the sides to keep it in place. A film will preferably be used where there is an issue of fibers being caught or stuck to the wound as in burn injuries.
Hospital and Health Care Facility attire: This can be in the form of a textile made from either a cotton/polyester or cotton based fabric where a percentage (can vary from 3% to 10%) of the yarn is treated cellulose or where the fibers of the yarn are a polymer in either filament or staple form. The article can be knit such as a cast lining or sock or can be woven such as a head cover or other article of clothing such as pajamas and underwear. The article can be used with no additional creams or medicines such as anti-biotic or steroidal salves or medicines. Such attire is especially useful for chronic patients or other bed-ridden patients in order to prevent and/or heal pressure and bed sores.
Bandages: These can be provided with an adhesive backing to keep them in place and can have a treatment pad made from a gauze using either polymeric or cellulose treated fibers. In addition, in some cases a treated film can be added to replace the gauze.
Bras: This can be made knit from either a cellulose or polymeric fiber which can include the water soluble cationic copper oxide particles
Nursing pads: This can be made from a series of absorbent layers which can contain loose fibers of either a polymer or treated cellulose mixed therein.
Padded adhesive: In some cases the layer incorporating the water insoluble cationic copper oxide may not be the first layer. The treated layer of textile can be the second or later layer (depending on the thickness of the pad) and will still be effective as long as liquid arrives at the treated layer.
Male and female underwear: For most treatments a gusset knit from either a treated polyester or mixed treated cellulose yarn will suffice.
Sheath for burn: These will preferably be made from the new breathable polymers that allow for the wicking and dispersion of moisture through them which polymers have been produced with the water insoluble cationic copper oxide particles introduced into the extrusion process. The film can be placed directly on the burn area and will reduce condensation on the wound surface while having the desired effect of the cationic copper.

While the invention will now be described in connection with certain preferred embodiments in the following examples and with reference to the attached figures so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments, will serve to illustrate the practice of this invention, it being understood that the particulars shown are by way of example and for purposes of illustrative discussion of preferred embodiments of the present invention only, and are presented in the cause of providing what is believed to be the most useful and readily understood description of formulation procedures, as well as of the principles and conceptual aspects of the invention.

In the drawings:
Figure 1a and Figure 1b are photographs of the top of a foot of a diabetic patient taken before and after treatment according to the present invention as described in example 3 hereinafter.
Figure 2a and Figure 2b are photographs of the sole of the foot of said diabetic patient taken before and after treatment according to the present invention as described in example 3 hereinafter
Figure 3a and Figure 3b are photographs of a lateral surface of the foot of a paraplegic patient taken before and after treatment according to the present invention as described in example 4 hereinafter.
Figure 4a and Figure 4b are photographs of the sole of the foot of a different diabetic patient taken before and after treatment according to the present invention as described in example 5 hereinafter.
Figure 5a and Figure 5b are photographs of a profile of a teenage patient suffering from acne taken before and after treatment according to the present invention as described in example 6 hereinafter.
Figure 6 is an electron microscope photograph of a polypropylene breathable film, which was prepared by introducing 1 % water insoluble copper oxide into the master batch before extrusion of the film, to form a film having microscopic water insoluble particles of ionic copper oxides in powdered form, embedded directly therein with a portion of said particles being exposed and protruding from surfaces thereof; and showing up as white dots in the electron microscope photograph thereof; and
Figure 7 is an electron microscope photograph of a polyester fiber prepared by introducing 1 % water insoluble copper oxide into the master batch before extrusion of the fiber, to form fibers having microscopic water insoluble particles of ionic copper oxides in powdered form, embedded directly therein with a portion of said particles being exposed and protruding from surfaces thereof, and showing up as white dots in the electron microscope photograph thereof;

### Example 1:Preparation of fabrics from treated cellulose fibers

1st. A cellulose fiber is chosen for the desired end use. Such fibers as Tencel, or acetate, or viscose or raw cotton are among the fibers that can be used. It is necessary to note that the fibers must be cellulose based as the plating will use the OH groups on the surface for initial attachment to the fiber. The length of the fiber chosen is a function of the end use and is common knowledge in the industry (i.e. long staple fibers are mixed with other fibers that have the same length such as in the case of combed cotton, etc.).
2nd. The fibers pass through the various chemical processes as described herein:
   1. Fibers are prepared in a thin mat to assure a deposition of the correct cationic species.
   2. The mat is soaked in a solution of Tin Dichloride and hydrochloric acid. The mat is allowed to soak for a small amount of time to insure complete absorption.
   3. The mat is then squeezed to remove almost all liquid and washed in water to assure the removal of all the tin solution.
   4. The mat is then placed in a very dilute solution of palladium dichloride and hydrochloric acid. While other metal salts can be used for this process, palladium was found to be the most efficient.
   5. After removal from the palladium dichloride the mat is once again washed and again squeezed to assure the removal of all extraneous liquid.
      At this point the mat will have changed color to a light tan.
   6. A chelated copper sulfate solution is prepared using copper sulfate, polyethylglycol, and EDTA. The pH of the solution is controlled by adding sodium hydroxide to the solution. A reductant is added to the copper sulfate solution. While many reductants can be used formaldehyde was chosen as the preferred compound.
   7. The mat is placed in the solution and allowed to go through the process which can take up to 7 minutes to occur. The mat must be squeezed or patted down during the plating process.
   8. The mat is then washed in water to remove excess dust and allowed to dry.
   9. At the end of the process, the fibers are plated with an ionic form of copper and have a dark brown mixed shade color.
   10. The fibers are blended with other fibers (the same untreated or other fibers) so that the end product contains only the amount of the desired copper oxide plated fibers. In some cases a 1% blend/99% other fibers is necessary and in other cases as much as 30% treated fibers/70% other fibers or any combination is prepared. This can be done in several ways all known to people familiar with the art of textile yarn spinning.
   11. The mixed fibers run through all normal textile processes, i.e. in the case of an open-end spun product: carding, sliver, spinning.
   12. Once yarn is obtained it can be either woven or knit depending on the desired end-use.
   13. Fabrics can be used as are or they can then be dyed or printed but not bleached, as this will cause the copper to disconnect itself from the cellulose substrate.
   14. The textile fabric can than be easily converted into the desired product.

### Example 2: Preparation of fabrics or films from treated Polymeric materials:

A 1. A polymeric material is chosen for the desired end use. Such fibers as polyester, polypropylene, polyethylene, nylon 66, nylon 6, etc. are among the fibers that can be used. The fiber can be formed into either a filament form or short staple form.
A 2. A master batch is prepared using the same base material as the desired yarn into which a copper oxide powder is added. For most textile end uses the master batch may have a 20% - 25% concentration of the copper oxide powder included in it. This master batch will be added to the polymer being extruded and diluted so that only about 1% or 2% of the material will be in the finished yarn. A certain amount of this copper will appear on the surface of a polymeric fiber and can be observed in an electron microscope picture.
A 3. If the fiber is a filament fiber it can be woven or knit to produce a textile.
A 4. If the fiber is a staple fiber it can be mixed with other fibers just the way the coated fibers described above are mixed and then follow the same process of manufacturing.
A 5. Once yarn has been completed, it can woven or knit into a textile product which follows the normal and accepted systems for finished product conversion.
B 1. A polymeric material is chosen for the desired end use. Such polymers as polyester, polypropylene, polyethylene, nylon 66, nylon 6, etc. are among the polymers that can be used. The polymeric material can be formed into either a film, or a sheath.
B2. A master batch is prepared using the same base material as the desired polymer into which a copper oxi de powder is added. For most end uses the master batch may have a 1-3% concentration of the copper oxide powder included in it. This master batch will be added to the polymer being extruded. A certain amount of this copper will appear on the surface of a polymeric film or sheath and can be observed in an electron microscope picture.

### Example 2 C- Preparation of fibers.

A total of 500 grams of a polyamide bi-component compound were prepared by heating the two beaded chemicals in separate baths each at 160 °C.

The two separate components were then mixed together and allowed to stir for 15 minutes until the mixture appeared to be homogenous in color.

The mixed chemistry was again divided into two separate pots. In one pot, 25 grams of a mixture of CuO and Cu₂O powder was added yielding a 1% mixture. In the second pot 6.25 grams of a mixture of CuO and Cu₂O were added yielding a 0.25% mixture. In both cases, the temperature of 160 °C was maintained. The compounds were stirred until they appeared homogenous in color.

The two mixtures were run through a spinneret with holes that yielded fibers of between 50 and 70 microns in diameter. Since the Cu++ releasing copper oxide powders were ground to particles of less than 20 microns no obstructions in the spinneret holes were observed. The extruded fibers were air-cooled and spun on to cones.

The resulting nylon fibers having Cu++ releasing copper oxide incorporated therein can be used in many of the appl i cations of the present invention including in bandages, in socks for diabetics, in gloves or socks for patients suffering from eczema or psoriasis or their hands or feet, etc.

As will now be understood by persons skilled in the art, the difference between the normal process of manufacturing any synthetic fiber and this process, is the addition of the Cu++ releasing copper oxide powders in the raw material s, and for many uses of the present invention such polymers as polyester, nylon and polypropylene can be interchangeably used.

### Example 3: Healing of ulcerative sores caused by diabetes

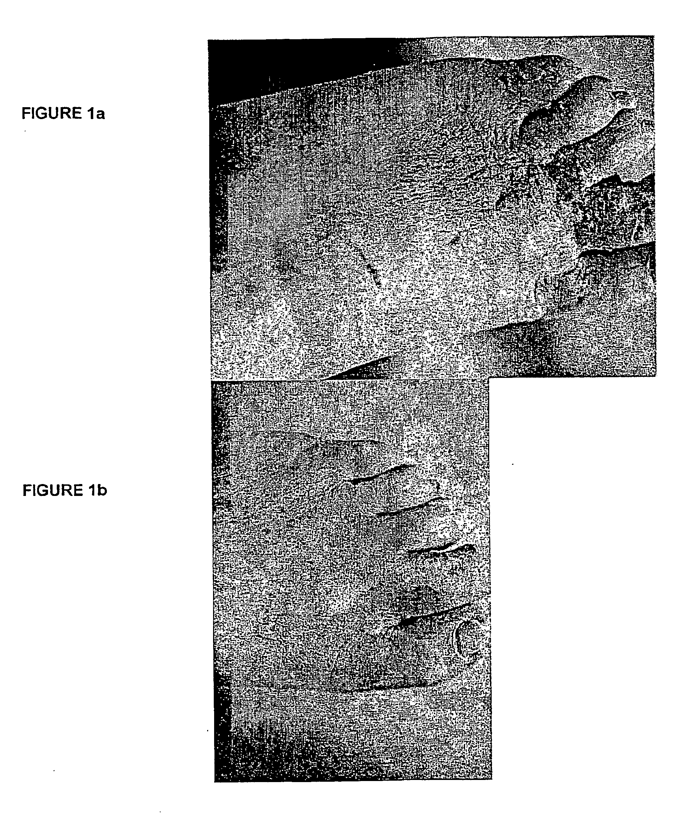

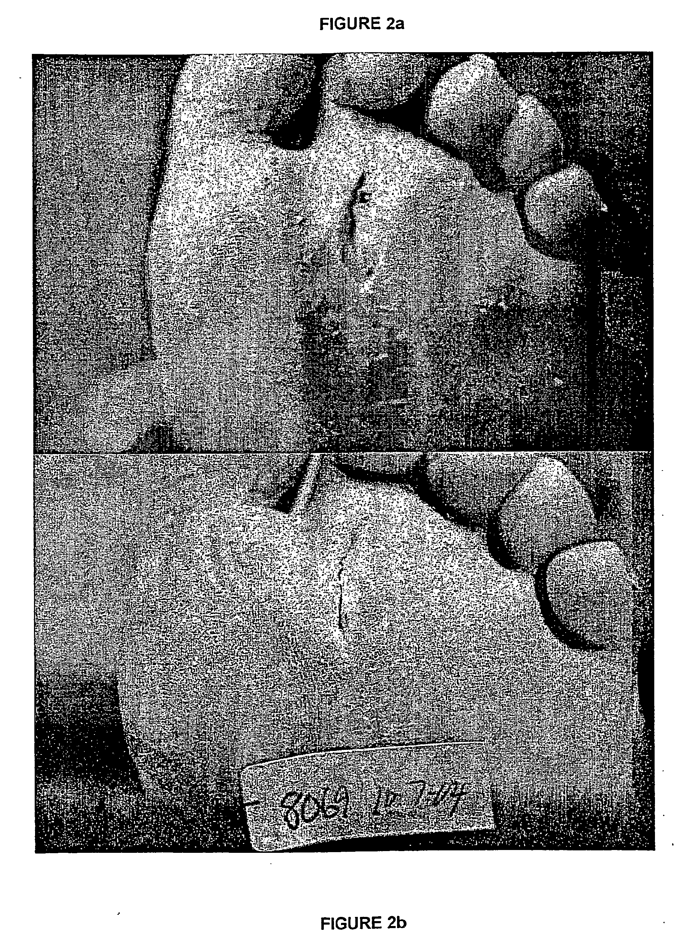

Referring to figures 1a and 2a, there are seen the top and sole of a 62 year old white female diabetic patient wherein on the sole of the foot there is seen an ulcerative sore which was 1.5 cm deep and which had already reached the bone, and therefore this patient was scheduled for amputation of this area of the foot two weeks from the date of September 30, 2004 upon which the photographs of figures 1 a and 2a were taken.

The doctor of this patient, who was assisting in clinical trials of the product of the present invention, wrapped the patient's foot with a gauze containing 3% cellulose fibers as prepared according to the method described in US 2003/0198945 (WO 03/086478) and as described hereinbefore, wherein said treated cellulose fibers are coated with ionic copper selected from the group consisting of Cu⁺ and Cu⁺⁺ ions in that formed on the surface of said fibers are insoluble copper oxide compounds of Cu⁺ and Cu⁺⁺.

As can be seen in figures 1b and 2b, which are photographs taken of the same foot of the same patient one week later on October 7, 2004, there resulted an amazing clearing of vascular lesions, regeneration of the dermal layer, and most amazingly, a cleaning and closure of the diabetic ulcer in the sole of the foot.

As a result of this treatment, amputation of the foot was no longer necessary.

### Example 4:Healing of paraplegic lesion sore

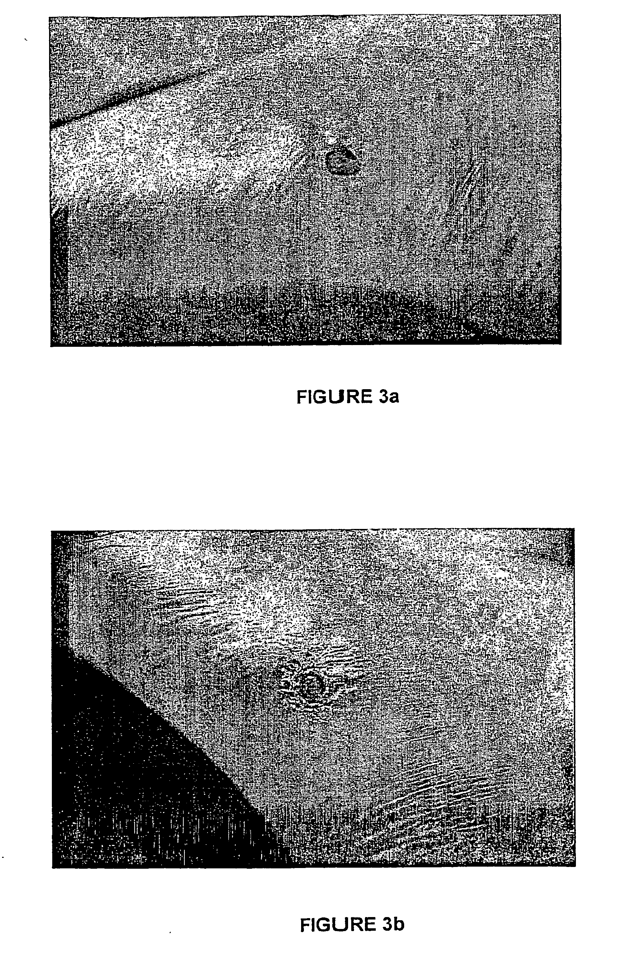

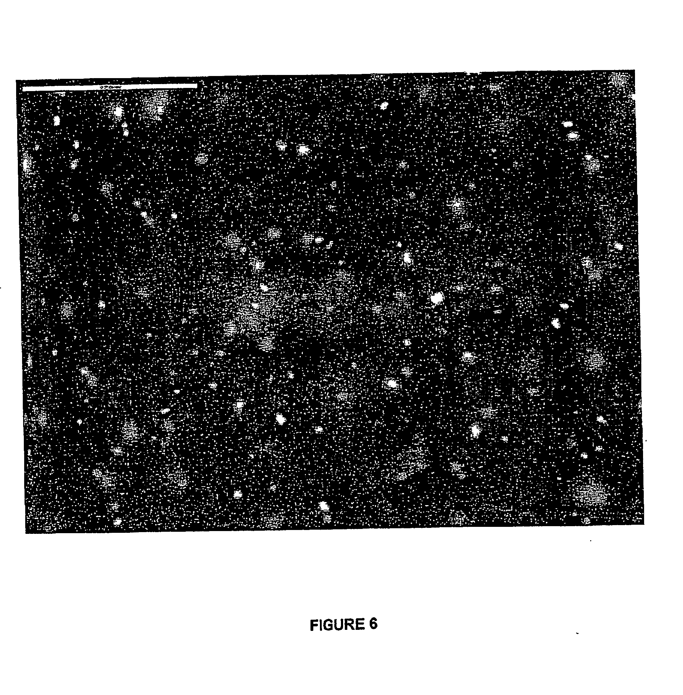

Referring to figure 3a, there is seen a photograph taken on September 23, 2004 of a lesion on the lateral surface of a foot of a 36 year old white male paraplegic who had this lesion for 6 months as a result of a sore from contact with the limb clamp of his wheelchair. On said date, the area was wrapped with a polypropylene breathable film which was prepared by introducing 1% water insoluble copper oxide into the master batch before extrusion of the film, to form a film having microscopic water insoluble particles of ionic copper oxides in powdered form, embedded directly therein with a portion of said particles being exposed and protruding from surfaces thereof as seen in figure 6 attached hereto, which particles release Cu⁺⁺.

Referring to figure 3b which is a photograph taken two weeks later on October 7, 2004, it can be seen that this lesion that had not responded to any conventional treatment over a six-month period, was beginning to heal and the regeneration of fibroblasts are clearly evident in the photograph.

### Example 5: Improvement of skin granulation of a diabetic

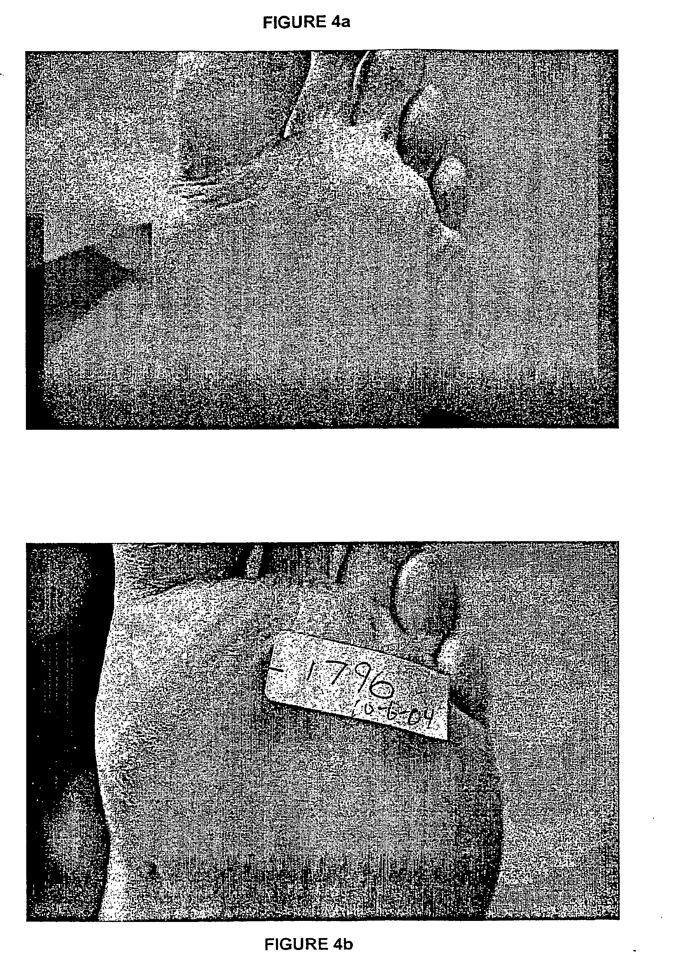

Referring to figure 4a, there is seen a photograph of the sole of a 76 year old white male diabetic patient, which photograph was taken on September 28, 2004, and wherein severe granulation of the skin area is seen.

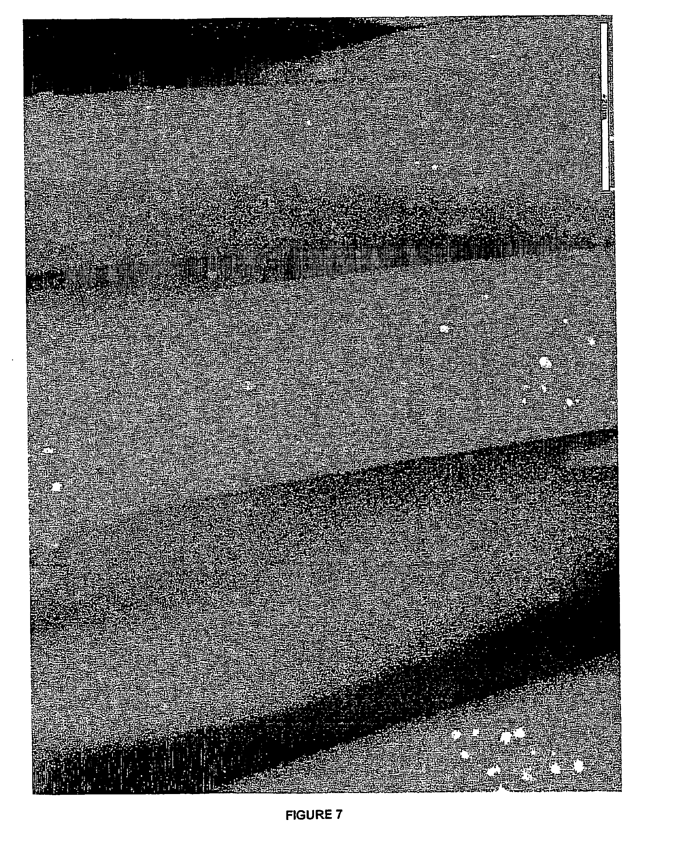

On September 28^{th}, this patient was instructed to begin wearing a specially prepared pair of socks which was made of polyester and in which there were introduced polyester fibers formed with Cu⁺⁺ releasing copper oxide powders, which fibers are shown in figure 7 and which fibers have microscopic water insoluble particles of ionic copper oxides in powdered form, embedded directly therein with a portion of said particles being exposed and protruding from surfaces thereof, which particles release Cu⁺⁺ to form a fabric containing 1 % copper oxide on the underside thereof.

Referring to figure 4b, which was a photograph taken on October 6, 2004, it can be seen that there has occurred a regeneration of the previously granulated skin area.

It is therefore believed that providing diabetics with socks prepared according to the present invention can serve to prevent the formation of diabetic granulation, ulcers, and lesions, and can also be used in the treatment thereof.

### Example 6: Treatment of teenage acne

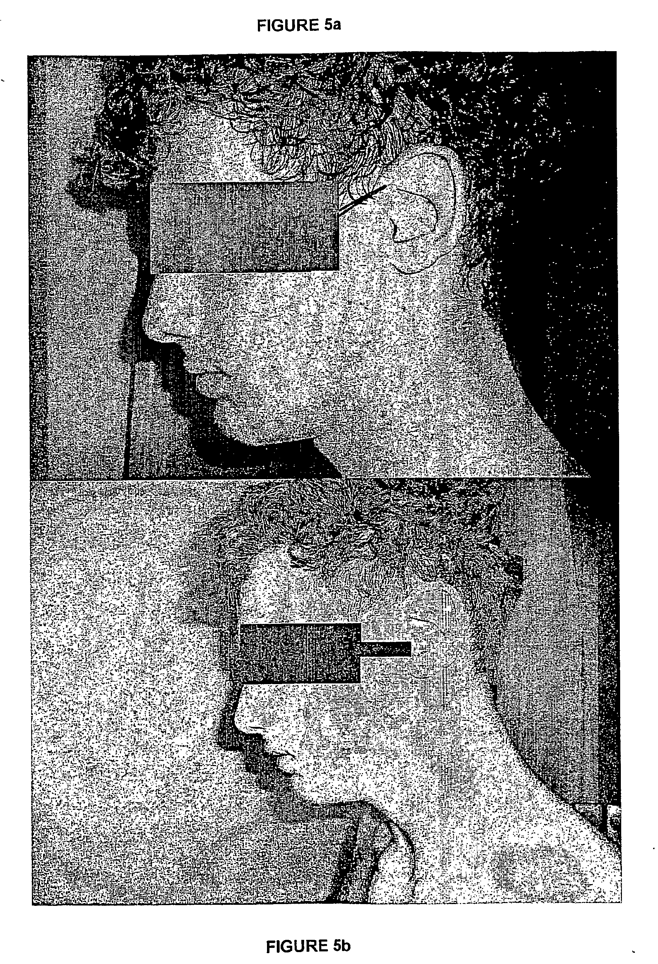

Referring to figure 5a, there is seen a photograph of the side view of a 16 year old white male suffering from acne which picture was taken on October 10, 2004.

This patient was instructed to place, each night, adjacent to the affected area, a gauze pad which was made of polyester and in which there were introduced polyester fibers formed with Cu⁺⁺ releasing copper oxide powders, which fibers are shown in figure 7 and which fibers have microscopic water insoluble particles of ionic copper oxides in powdered form, embedded directly therein with a portion of said particles being exposed and protruding from surfaces thereof, which particles release Cu⁺⁺ to form a gauze pad containing 1 % copper oxide.

Referring to figure 5b, there is seen a photograph taken of this patient on October 13^{th}, after only 3 nights of usage of the gauze pad according to the present invention, and already a vast improvement and decrease in the size of the acne sores was visible, which decrease normally occurs only after several weeks.

### Example 7: Scarless healing of blistered burn

A white female 57 year old woman suffered a second degree blistered burn on her upper thigh which was approximately 6 in. by 2 in. in size, as a result of a scalding glass of tea knocked into her lap.

The burn area was covered with a gauze pad according to the present invention which was made of cotton and in which there were introduced cellulosic fibers which were formed with Cu⁺⁺ releasing copper oxide powders, which fibers were woven into the gauze to form a final product which was 97% cotton and 3% cationic copper releasing fibers. The gauze pad was periodically replaced with fresh pads of the same material for a period of three weeks after which the pad was removed to reveal an area from which the blistered and burnt skin had totally sloughed off leaving a fresh layer of scar-free epidermal tissue, which area one week later was substantially indistinguishable from the surrounding area.

### Example 8: Clinical Testing

Dr. Michael S. Smith, a Board Certified Neurologist with a Master's Degree in Experimental Statistics, was asked to analyze the effectiveness of socks prepared with a lower panel of fabric incorporating water-insolubie copper compounds which release Cu⁺ and Cu⁺⁺ ions, upon a variety of podiatric conditions, i.e. erythema, itching and burning, scaling, vesicular eruptions, fissuring, drainage, odor and edema.

One group of patients was studied; and the results were compared to the experience the podiatrist had with patients with similar conditions who were not treated with socks according to the present invention

### RESULTS:

The following results are all considered statistically significant, meaning that there is credible medical evidence that treatment with the socks according to the present invention is effective in the period of follow up studied, since the confidence interval of all the results did not include 0 and the p-values for all results were <0.001.

### Demographics:

There were 56 patients in all, 17 women and 39 men. The average age of the group was 58 with a standard deviation of 16 years (range 21-85 years). Twenty-one (21) were diabetic, 21 were older than 65, and 24 were followed more than one time.

### Variables:

Measures were studied: burning and itching, vesicular eruptions, fissuring, "Long range follow-up referred to having been evaluated more than one time after use of the socks. There was a three level ordinal scale used: present, improvement, and resolved. Movement along this scale (from "present" to "improved" or from either of the first two to "resolved") was considered a positive sign, movement the other way (from "improved" to "present") considered a negative sign. If a patient was considered resolved on the first visit after wearing the socks, that individual could at best be scored a "same" for long-term follow-up. Therefore, "same" could be equally considered to be "holding improvement". The average length of time in the long term section was defined as being the time between the first visit and the date when the last comment was made about the patient. Only patients who had a specific problem at the outset of the study were counted later. In no instance, did a patient who had no specific problem develop one. In the instances of edema, odor, and drainage, the sample sizes were too small to draw any conclusions, although the results were tabulated.

### Example 8C: Fissuring (37 patients):

C1. All 37 patients improved; 15 (40%) resolved completely with an average follow-up of 10 days, 95% CI (0.25, 0.58). This is highly significant.

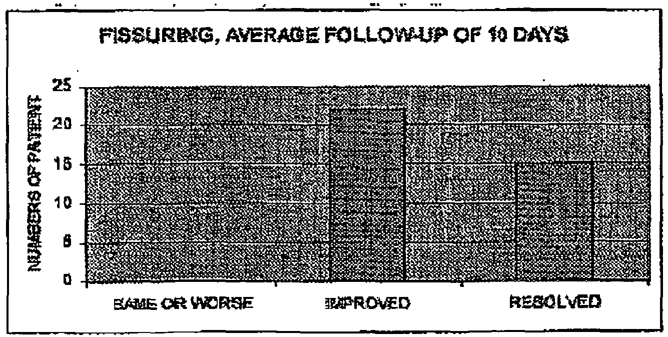

C2. Longer term study (17 patients):

All 17 patients improved, 6 (35%) resolved completely in an average follow-up of 39 days, 95% CI (0.14, 0.62). Again, diabetics and elderly shared in the improvement.

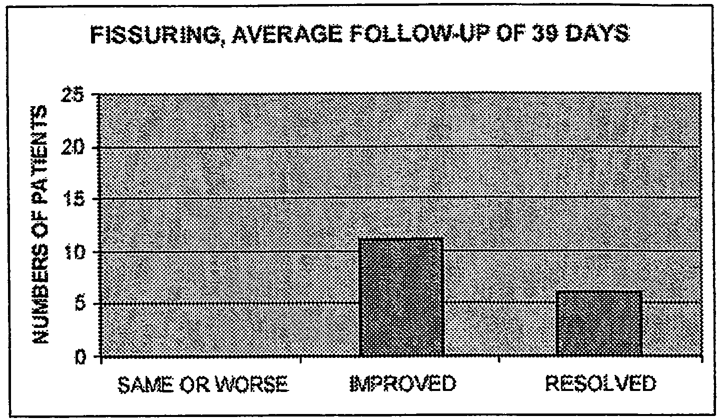

### Example 8D: Burning Or Itching (23 patients):

D1. Nineteen of the 23 improved (83%), four stayed the same or reverted in the average follow-up period of 8 days. The 95% confidence interval is (0.61, 0.95) with a p-value of 0.003, again highly significant.

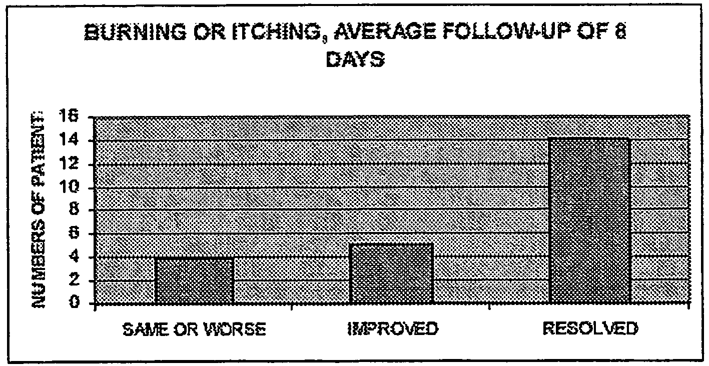

D2. Longer term study (8 patients):

All 8 patients were unchanged over an average follow-up of 46 days, meaning that their initial improvement was maintained. The numbers were too small to study diabetics and elderly.

### Example 8E: Vesicular eruptions (23 patients):

E1. All 23 patients improved; 13 (56%) resolved completely, 95% CI (0.34, 0.76).

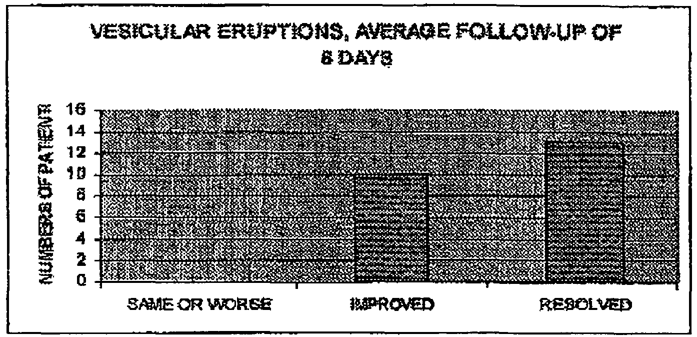

E2. Longer term study (10 patients):

All 10 patients maintained their improvement or resolved (6) over an average follow-up of 45 days. The proportions were similar for both diabetics and patients over the age of 65.

### DISCUSSION:

The purpose of the study was to see if patients with a variety of podiatric ailments would improve only by wearing socks having a fabric panel according to the present invention. One issue in the treatment of the above conditions is compliance in obtaining and using the treatment (special socks). A related issue is the proper application of treatment (special socks) on the plantar aspect of the feet and in the interdigital areas.
1. For purposes of these examples the following was assumed in the analysis of the data provided:
   a. The patients were a reasonable, representative sample of the population of patients with these conditions. There was no information received that would contradict this assumption. There were men, women, elderly, young, diabetic, and non-diabetic patients.
   b. The patients were independent of one another; that is, the selection of one individual had no effect upon the selection of another.
   c. The definitions of improvement and resolution were constant for each patient.
   d. The sample size was known and appropriate to perform analysis.
   e. Outcomes could be defined as dichotomous.
   The presence of these assumptions allowed a binomial probability distribution to be used.
2. There was no control group reported; however, information was received stating that the podiatrist believed it unlikely that any patient would have resolved or improved in the time frame of the study only by wearing his or her regular socks. Given such information, all of the above results, would be considered statistically significant, meaning that there is medical evidence that treatment with fabric panels according to the present invention is effective in this period of follow-up.
   It is important to understand the vocabulary used in describing the study:
   - *Population:* the group about which one wishes to learn. In this instance, the population are all patients with the above listed foot conditions.
   - *Sample:* a subset of a population.
   - *Random sample:* A subset chosen where each member of the population has a defined, non-zero probability of being chosen.
   - *Parameter:* a numerical measure of the population.
   - *Statistic:* a numerical measure of the sample.
   - *p-value:* the probability that we would obtain the specific sample statistic (or one more extreme) if the null hypothesis (hypothesis of no change) were true. In the context of this study, a p-value of less than 0.001 means that the probability of obtaining these results by chance alone is less than 1 in 1000. Typically, 1 in 20 is considered the "cut-off" point. Minitab software does not compute p-values to four decimal places, so many of the values obtained here are even smaller.
   - *A confidence interval* contains a range of plausible values for the parameter. We call it a confidence interval, because while unknown, the parameter does exist, and the interval either contains or does not contain the parameter. It is NOT a probability question. For this study, if we assume that no patient would improve in the time frame studied with conventional treatment then so long as the interval does not contain 0, the results are significant, since no plausible value of the parameter is 0. If some other proportion were postulated for improvement, then any interval that did not contain that particular value would be considered significant. In this study, with the above information, all areas reached statistical significance.
   - *Circle of inference:* We sample from a population, obtain a result (a statistic), and use that value to infer something about a parameter which is part of a population.

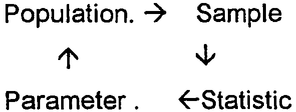

It is important to recognize that one can seldom identify all mem bers of a population, so that its numerical measure, a parameter, remains unknown.

For this study, since we cannot know all members of the population, the result of the sample, the proportion improved (or resolved), is used to say a similar proportion of the population would be improved as well. If the sample is appropriately chosen, then the estimate has value. We must realize, of course, that other samples would lead to other results, so that there is a *range* of plausible values that samples could conceivably have, and our sample result was one of those potential values, as described above.

### CONCLUSION:

Compared to historical controls, patients with socks prepared with a lower panel of fabric incorporating water-insoluble copper compounds which release Cu⁺ and Cu⁺⁺ ions as according to the present invention, had significant improvement or resolution in the following conditions:

| | |
|---|---|
| | Vesicular eruptions |
| Burning/itching | Fissures |

Moreover, since nearly 40% (19 of 51) of the group was either diabetic or older than 65 (10 were both diabetic and older than 65), this study is statistically significant for improvement or resolved for all the above conditions for people with diabetes, including elderly diabetics.

Itching and burning are both healing issues.

Vesicular eruptions is an eruption of capillaries that are close to the surface of the skin and is thus also a healing issue.

Fissuring is a break in the skin usually where it joins a mucous membrane producing a crack-like sore or ulcer and this is also a healing issue which can be dealt with according to the present invention.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A wound-healing material which comprises a polymeric fiber, ligament, film, sheet or sheath having embedded directly therein microscopic particles of copper (I) oxide and/or copper (II) oxide in powdered form which release Cu⁺ ions, Cu⁺⁺ ions or combinations thereof upon contact with a fluid, with a portion of said particles being exposed and protruding from surfaces thereof, or comprises a cellulosic fiber plated with the copper oxide, for use in bringing in contact with a body surface having wounds selected from sores, abrasions, ulcerations, lesions, cutaneous openings or burns for the treatment and healing thereof.

2. The wound-healing material for use according to claim 1, wherein the material is a bandage comprising fibers incorporating the copper (I) oxide and/or copper (II) oxide, for the treatment of wounds selected from sores, cold sores, cutaneous openings, ulcerations, lesions, abrasions and burns.

3. The wound-healing material for use according to claim 2, wherein said bandage is formed of a gauze material having said copper (I) oxide and/or copper (II) oxide incorporated therein.

4. The wound-healing material for use according to claim 1, wherein the material is a bandage comprising a polymeric film having microscopic particles of the copper (I) oxide and/or copper (II) oxide in powdered form embedded directly therein with a portion of said particles being exposed and protruding from surfaces thereof, for the treatment of wounds selected from sores, cold sores, cutaneous openings, ulcerations, lesions, abrasions and burns.

5. The wound-healing material for use according to claim 1, wherein the material is patient attire for hospital and health care facilities comprising fibers incorporating the copper (I) oxide and/or copper (II) oxide for preventing the formation of wounds selected from bed sores.

6. The wound-healing material for use according to claim 1, wherein the material is a protective sheath for a body limb, comprising a polymeric film having microscopic particles of the copper (I) oxide and/or copper (II) oxide in powdered form embedded directly therein with a portion of said particles being exposed and protruding from surfaces thereof, for the treatment of wounds selected from sores forming on a body limb.

7. The wound-healing material for use according to claim 1, wherein the material is a protective sheath for a body limb, comprising fibers incorporating the copper (I) oxide and/or copper (II) oxide, for the treatment of wounds selected from sores forming on a body limb.

8. The wound-healing material for use according to claim 1, wherein the material is a dressing comprising fibers incorporating the copper (I) oxide and/or copper (II) oxide, for the treatment of wounds selected from sores, cold sores, cutaneous openings, ulcerations, lesions, abrasions and burns.

9. The wound-healing material for use according to claim 1, wherein the material is a dressing comprising a polymeric film having microscopic particles of the copper (I) oxide and/or copper (II) oxide in powdered form embedded directly therein with a portion of said particles being exposed and protruding from surfaces thereof, for the treatment of wounds selected from sores, cold sores, cutaneous openings, ulcerations, lesions, abrasions and burns.

10. The wound-healing material for use according to claim 1, wherein the material is underpants for men comprising fibers incorporating the copper (I) oxide and/or copper (II) oxide, for treating the outbreak of wounds selected from male genital herpes sores.

11. The wound-healing material for use according to claim 1, wherein the material is bras and nursing pads for nursing mothers comprising fibers incorporating the copper (I) oxide and/or copper (II) oxide, for the treatment of wounds selected from nipple sores.

12. The wound-healing material for use according to claim 1, wherein the material is a dressing comprising fibers incorporating the copper (I) oxide and/or copper (II) oxide, for the treatment of wounds selected from acne sores.

13. The wound-healing material for use according to claim 12, wherein said fibers are incorporated into the pad of a padded adhesive bandage.

14. The wound-healing material for use according to claim 1, wherein the material is a fabric comprising fibers incorporating the copper (I) oxide and/or copper (II) oxide, for bringing in contact with a body surface affected by psoriasis for the treatment thereof.

15. The wound-healing material for use according to claim 1, wherein the material is a fabric comprising fibers incorporating the copper (I) oxide and/or copper (II) oxide, for bringing in contact with a body surface affected by wounds selected from eczema for the treatment thereof.

16. The wound-healing material for use according to claim 1, wherein the material is a fabric comprising fibers incorporating the copper (I) oxide and/or copper (II) oxide, for bringing in contact with a body surface having wounds selected from sores, abrasions and burns for the treatment and healing thereof.

17. The wound-healing material for use according to claim 1, wherein the material is a suturing material comprising fibers incorporating the copper (I) oxide and/or copper (II) oxide.

18. The wound-healing material for use according to claim 1, wherein the material is a suturing material comprising polymeric filament having microscopic particles of the copper (I) oxide and/or copper (II) oxide in powdered form embedded directly therein with a portion of said particles being exposed and protruding from surfaces thereof.

19. The wound-healing material for use according to claim 1, wherein the material is a wound-healing fabric to be used in a military uniform or article of inner or outer clothing, said fabric including fibers incorporating the copper (I) oxide and/or copper (II) oxide which upon entry into a wound of a military personnel sustaining a wound while wearing the same, achieves both an anti-bacterial effect and a healing effect on said wound.

20. The wound-healing material for use according to claim 1, wherein the material is a military uniform or article of inner or outer clothing comprising fibers incorporating the copper (I) oxide and/or copper (II) oxide, which fibers upon entry into a wound of a military personnel sustaining a wound while wearing the same, achieves both an anti-bacterial effect and a healing effect on said wound.

21. The wound-healing material for use according to claim 1, wherein the material is a wound-healing fabric or an extruded wound-healing film, filament or sheath comprising the copper (I) oxide and/or copper (II) oxide, for bringing in contact with a body surface having wounds selected from sores, abrasions, ulcerations, lesions, cutaneous openings or burns for the treatment and healing thereof.

22. The wound-healing material for use according to claim 1, wherein the material is a garment selected from the group consisting of pyjamas, nightgowns and underwear for patient attire for hospital and health care facilities, said garment having a panel including the copper (I) oxide and/or copper (II) oxide incorporated at least in the area of the garment which lies adjacent to the buttocks area of a patient, for the prevention and healing of wounds selected from bed and pressure sores.

23. The wound-healing material for use according to any of claims 2, 5, 7, 8, 10-12, 14, 15 and 20, wherein said fibers are polymeric fibers having said microscopic particles of the copper oxide in powdered form embedded directly therein with a portion of said particles being exposed and protruding from surfaces thereof.

24. The wound-healing material for use according to any of claims 2, 5, 7, 8, 10-12, 14, 15 and 20, wherein said fibers are cellulosic fibers plated with the copper oxide.

## Patentansprüche

1. Wundheilungsmaterial, das eine polymere Faser, ein polymeres Filament, eine polymere Dünnfolie, Dickfolie oder Umhüllung umfasst, in die/das mikroskopische Teilchen von Kupfer(I)oxid und/oder Kupfer(II)oxid in Pulverform direkt eingebettet sind, das Cu⁺-Ionen, Cu⁺⁺-Ionen oder Kombinationen davon auf den Kontakt mit einem Fluid hin freigibt, wobei ein Teil der Teilchen bloßgelegt ist und aus den Oberflächen davon herausragt, oder eine Cellulosefaser umfasst, die mit dem Kupferoxid überzogen ist, zur Verwendung zum Inkontaktbringen mit einer Körperoberfläche, die Wunden aufweist ausgewählt unter wunden Stellen, Hautabschürfungen, Geschwüren, Läsionen, Hautöffnungen oder Verbrennungen, zur Behandlung und Heilung derselben.

2. Wundheilungsmaterial zur Verwendung nach Anspruch 1, wobei das Material eine Binde ist, die Fasern umfasst, in die das Kupfer(I)oxid und/oder Kupfer(II)oxid integriert ist/sind, zur Behandlung von Wunden ausgewählt unter wunden Stellen, kalten Wunden, Hautöffnungen, Geschwüren, Läsionen, Hautabschürfungen und Verbrennungen.

3. Wundheilungsmaterial zur Verwendung nach Anspruch 2, wobei die Binde aus einem Verbandmullmaterial gebildet ist, in das das Kupfer(I)oxid und/oder Kupfer(II)oxid integriert ist/sind.

4. Wundheilungsmaterial zur Verwendung nach Anspruch 1, wobei das Material eine Binde ist, die eine polymere Dünnfolie umfasst, in die mikroskopische Teilchen des Kupfer(I)oxids und/oder Kupfer(II)oxids in Pulverform direkt eingebettet sind, wobei ein Teil der Teilchen bloßgelegt ist und aus den Oberflächen davon herausragt, zur Behandlung von Wunden ausgewählt unter wunden Stellen, kalten Wunden, Hautöffnungen, Geschwüren, Läsionen, Hautabschürfungen und Verbrennungen.

5. Wundheilungsmaterial zur Verwendung nach Anspruch 1, wobei das Material Patientenbekleidung für Krankenhaus- und Gesundheitspflegeeinrichtungen ist, die Fasern umfasst, in die das Kupfer(I)oxid und/oder Kupfer(II)oxid integriert ist/sind, zum Verhindern der Bildung von Wunden ausgewählt unter wundgelegenen Stellen.

6. Wundheilungsmaterial zur Verwendung nach Anspruch 1, wobei das Material eine Schutzumhüllung für ein Körperglied ist, die eine polymere Dünnfolie umfasst, in die mikroskopische Teilchen des Kupfer(I)oxids und/oder Kupfer(II)oxids in Pulverform direkt eingebettet sind, wobei ein Teil der Teilchen bloßgelegt ist und aus den Oberflächen davon herausragt, zur Behandlung von Wunden ausgewählt unter an einem Körperglied gebildeten wunden Stellen.

7. Wundheilungsmaterial zur Verwendung nach Anspruch 1, wobei das Material eine Schutzhülle für ein Körperglied ist, die Fasern umfasst, in die das Kupfer(I)oxid und/oder Kupfer(II)oxid integriert ist/sind, zur Behandlung von Wunden ausgewählt unter an einem Körperglied gebildeten wunden Stellen.

8. Wundheilungsmaterial zur Verwendung nach Anspruch 1, wobei das Material ein Verband ist, der Fasern umfasst, in die das Kupfer(I)oxid und/oder Kupfer(II)oxid integriert ist/sind, zur Behandlung von Wunden ausgewählt unter wunden Stellen, kalten Wunden, Hautöffnungen, Geschwüren, Läsionen, Hautabschürfungen und Verbrennungen.

9. Wundheilungsmaterial zur Verwendung nach Anspruch 1, wobei das Material ein Verband ist, der eine polymere Dünnfolie umfasst, in die mikroskopische Teilchen des Kupfer(I)oxids und/oder Kupfer(II)oxids in Pulverform direkt eingebettet sind, wobei ein Teil der Teilchen bloßgelegt ist und aus den Oberflächen davon herausragt, zur Behandlung von Wunden ausgewählt unter wunden Stellen, kalten Wunden, Hautöffnungen, Geschwüren, Läsionen, Hautabschürfungen und Verbrennungen.

10. Wundheilungsmaterial zur Verwendung nach Anspruch 1, wobei das Material eine Unterhose für Männer ist, die Fasern umfasst, in die das Kupfer(I)oxid und/oder Kupfer(II)oxid integriert ist/sind, zum Behandeln des Ausbrechens von Wunden ausgewählt unter Herpesgeschwüren an den männlichen Genitalien.

11. Wundheilungsmaterial zur Verwendung nach Anspruch 1, wobei das Material Büstenhalter und Stilleinlagen für stillende Mütter ist, die Fasern umfassen, in die das Kupfer(I)oxid und/oder Kupfer(II)oxid integriert ist/sind, zur Behandlung von Wunden ausgewählt unter wunden Brustwarzen.

12. Wundheilungsmaterial zur Verwendung nach Anspruch 1, wobei das Material ein Verband ist, der Fasern umfasst, in die das Kupfer(I)oxid und/oder Kupfer(II)oxid integriert ist/sind, zur Behandlung von Wunden ausgewählt aus Akneentzündungen.

13. Wundheilungsmaterial zur Verwendung nach Anspruch 12, wobei die Fasern in die Wattierung eines wattierten Wundschnellverbands integriert sind.

14. Wundheilungsmaterial zur Verwendung nach Anspruch 1, wobei das Material ein Textilstoff ist, der Fasern umfasst, in die das Kupfer(I)oxid und/oder Kupfer(II)oxid integriert ist/sind, zum Inkontaktbringen mit einer Körperoberfläche, die von Schuppenflechte befallen ist, zum Behandeln derselben.

15. Wundheilungsmaterial zur Verwendung nach Anspruch 1, wobei das Material ein Textilstoff ist, der Fasern umfasst, in die das Kupfer(I)oxid und/oder Kupfer(II)oxid integriert ist/sind, zum Inkontaktbringen mit einer Körperoberfläche, die von Wunden befallen ist ausgewählt unter Hautekzem, für die Behandlung desselben.

16. Wundheilungsmaterial zur Verwendung nach Anspruch 1, wobei das Material ein Textilstoff ist, der Fasern umfasst, in die das Kupfer(I)oxid und/oder Kupfer(II)oxid integriert ist/sind, zum Inkontaktbringen mit einer Körperoberfläche, die Wunden aufweist ausgewählt unter wunden Stellen, Hautabschürfungen und Verbrennungen, zur Behandlung und Heilung derselben.

17. Wundheilungsmaterial zur Verwendung nach Anspruch 1, wobei das Material ein Nahtmaterial ist, das Fasern umfasst, in die das Kupfer(I)oxid und/oder Kupfer(II)oxid integriert ist/sind.

18. Wundheilungsmaterial zur Verwendung nach Anspruch 1, wobei das Material ein Nahtmaterial ist, das polymeres Filament umfasst, in das mikroskopische Teilchen des Kupfer(I)oxids und/oder Kupfer(II)oxids in Pulverform direkt eingebettet sind, wobei ein Teil der Teilchen bloßgelegt ist und aus den Oberflächen davon herausragt.

19. Wundheilungsmaterial zur Verwendung nach Anspruch 1, wobei das Wundheilungsmaterial ein Wundheilungstextilstoff ist, der in einer Militäruniform oder einem Artikel von Unter- oder Oberbekleidung verwendet werden soll, wobei der Textilstoff Fasern umfasst, in die das Kupfer(I)oxid und/oder Kupfer(II)oxid integriert ist/sind, das/die auf den Eintritt in eine Wunde von Militärpersonal, das eine Wunde erleidet, während es dieselbe trägt, sowohl eine antibakterielle Wirkung als auch eine Heilungswirkung bei der Wunde erreicht/erreichen.

20. Wundheilungsmaterial zur Verwendung nach Anspruch 1, wobei das Material eine Militäruniform oder ein Artikel von Unter- oder Oberbekleidung ist, die/der Fasen umfasst, in die das Kupfer(I)oxid und/oder Kupfer(II)oxid integriert ist/sind, welche Fasern auf den Eintritt in eine Wunde von Militärpersonal, das eine Wunde erleidet, während es dieselbe trägt, sowohl eine antibakterielle Wirkung als auch eine Heilungswirkung bei der Wunde erreichen.

21. Wundheilungsmaterial zur Verwendung nach Anspruch 1, wobei das Material ein Wundheilungstextilstoff oder eine extrudierte Wundheilungsdünnfolie, ein extrudiertes Wundheilungsfilament oder eine extrudierte Wundheilungsumhüllung ist, die/das das Kupfer(I)oxid und/oder Kupfer(II)oxid umfasst, zum Inkontaktbringen mit einer Körperoberfläche, die Wunden aufweist ausgewählt unter wunden Stellen, Hautabschürfungen, Geschwüren, Läsionen, Hautöffnungen oder Verbrennungen, zur Behandlung und Heilung derselben.

22. Wundheilungsmaterial zur Verwendung nach Anspruch 1, wobei das Material ein Kleidungsstück ist ausgewählt aus der Gruppe bestehend aus Schlafanzügen, Nachthemden und Unterkleidung für Patientenbekleidung für Krankenhaus- und Gesundheitspflegeeinrichtungen, wobei das Kleidungsstück eine Stoffbahn aufweist, die das Kupfer(I)oxid und/oder Kupfer(II)oxid umfasst und mindestens in den Bereich des Kleidungsstücks integriert ist, der neben dem Gesäßbereich eines Patienten liegt, zur Verhinderung und Heilung von Wunden ausgewählt unter wundgelegenen Stellen und Druckwunden.

23. Wundheilungsmaterial zur Verwendung nach einem der Ansprüche 2, 5, 7, 8, 10-12, 14, 15 und 20, wobei die Fasern polymere Fasern sind, in die mikroskopische Teilchen des Kupferoxids in Pulverform direkt eingebettet sind, wobei ein Teil der Teilchen bloßgelegt ist und aus den Oberflächen davon herausragt.

24. Wundheilungsmaterial zur Verwendung nach einem der Ansprüche 2, 5, 7, 8, 10-12, 14, 15 und 20, wobei die Fasern Cellulosefasern sind, die mit dem Kupferoxid überzogen sind.

## Revendications

1. Matériau pour la guérison des blessures qui comprend une fibre, un filament, un film, une feuille ou une gaine polymère ayant directement enchâssé à l'intérieur des particules microscopiques d'oxyde de cuivre (I) et/ou d'oxyde de cuivre (II) sous une forme pulvérulente qui libèrent des ions Cu⁺, des ions Cu⁺⁺ ou leurs combinaisons au contact d'un fluide, avec une partie desdites particules qui est exposée et qui fait saillie de leurs surfaces, ou qui comprend une fibre cellulosique plaquée d'oxyde de cuivre pour l'utilisation par mise en contact avec une surface corporelle présentant des blessures sélectionnées parmi les plaies, les exulcérations, les ulcérations, les lésions, les ouvertures cutanées ou les brûlures pour leur traitement et leur guérison.

2. Matériau pour la guérison des blessures pour l'utilisation selon la revendication 1, dans lequel le matériau est un bandage comprenant des fibres incorporant l'oxyde de cuivre (I) et/ou l'oxyde de cuivre (II), pour le traitement des blessures sélectionnées parmi les plaies, les boutons de fièvres, les ouvertures cutanées, les ulcérations, les lésions, les exulcérations et les brûlures.

3. Matériau pour la guérison des blessures pour l'utilisation selon la revendication 2, dans lequel ledit bandage est formé d'un matériau de type gaze ayant ledit oxyde de cuivre (I) et/ou oxyde de cuivre (II) incorporé(s) à l'intérieur.

4. Matériau pour la guérison des blessures pour l'utilisation selon la revendication 1, dans lequel le matériau est un bandage comprenant un film polymère ayant des particules microscopiques d'oxyde de cuivre (I) et/ou d'oxyde de cuivre (II) sous une forme pulvérulente enchâssées directement dedans avec une partie desdites particules qui est exposée et qui fait saillie de leurs surfaces, pour le traitement des blessures sélectionnées parmi les plaies, les boutons de fièvres, les ouvertures cutanées, les ulcérations, les lésions, les exulcérations et les brûlures.

5. Matériau pour la guérison des blessures pour l'utilisation selon la revendication 1, dans lequel le matériau est un vêtement hospitalier ou d'installations de soins de santé pour les patients comprenant des fibres incorporant l'oxyde de cuivre (I) et/ou l'oxyde de cuivre (II) pour prévenir la formation des blessures sélectionnées parmi les plaies de lit.

6. Matériau pour la guérison des blessures pour l'utilisation selon la revendication 1, dans lequel le matériau est une gaine protectrice pour un membre, comprenant un film polymère ayant des particules microscopiques d'oxyde de cuivre (I) et/ou d'oxyde de cuivre (II) sous une forme pulvérulente enchâssées directement à l'intérieur avec une partie desdites particules étant exposée et faisant saillie de leurs surfaces, pour le traitement des blessures sélectionnées parmi les plaies se formant sur un membre.

7. Matériau pour la guérison des blessures pour l'utilisation selon la revendication 1, dans lequel le matériau est une gaine protectrice pour un membre, comprenant des fibres incorporant l'oxyde de cuivre (I) et/ou l'oxyde de cuivre (II), pour le traitement des blessures sélectionnées parmi les plaies se formant sur un membre.

8. Matériau pour la guérison des blessures pour l'utilisation selon la revendication 1, dans lequel le matériau est un pansement comprenant des fibres incorporant l'oxyde de cuivre (I) et/ou l'oxyde de cuivre (II), pour le traitement des blessures sélectionnées parmi les plaies, les boutons de fièvre, les ouvertures cutanées, les ulcérations, les lésions, les exulcérations et les brûlures.

9. Matériau pour la guérison des blessures pour l'utilisation selon la revendication 1, dans lequel le matériau est un pansement comprenant un film polymère ayant des particules microscopiques d'oxyde de cuivre (I) et/ou d'oxyde de cuivre (II) sous une forme pulvérulente enchâssées directement à l'intérieur avec une partie desdites particules qui est exposée et qui fait saillie de leurs surfaces, pour le traitement des blessures sélectionnées parmi les plaies, les boutons de fièvre, les ouvertures cutanées, les ulcérations, les lésions, les exulcérations et les brûlures.

10. Matériau pour la guérison des blessures pour l'utilisation selon la revendication 1, dans lequel le matériau est un caleçon pour homme comprenant des fibres incorporant l'oxyde de cuivre (I) et/ou l'oxyde de cuivre (II), pour le traitement d'une flambée de blessures sélectionnées parmi les plaies d'herpès génital chez l'homme.

11. Matériau pour la guérison des blessures pour l'utilisation selon la revendication 1, dans lequel le matériau est un soutien-gorge et des compresses d'allaitement pour les mères allaitantes comprenant des fibres incorporant l'oxyde de cuivre (I) et/ou l'oxyde de cuivre (II), pour le traitement des blessures sélectionnées parmi les plaies du mamelon.

12. Matériau pour la guérison des blessures pour l'utilisation selon la revendication 1, dans lequel le matériau est un pansement comprenant des fibres incorporant l'oxyde de cuivre (I) et/ou l'oxyde de cuivre (II), pour le traitement des blessures sélectionnées parmi les plaies acnéiques.

13. Matériau pour la guérison des blessures pour l'utilisation selon la revendication 12, dans lequel lesdites fibres sont incorporées dans la compresse d'un bandage adhésif rembourré.

14. Matériau pour la guérison des blessures pour l'utilisation selon la revendication 1, dans lequel le matériau est un textile comprenant des fibres incorporant l'oxyde de cuivre (I) et/ou l'oxyde de cuivre (II), pour la mise en contact avec une surface corporelle affectée d'un psoriasis pour le traiter.

15. Matériau pour la guérison des blessures pour l'utilisation selon la revendication 1, dans lequel le matériau est un textile comprenant des fibres incorporant l'oxyde de cuivre (I) et/ou l'oxyde de cuivre (II), pour la mise en contact avec une surface corporelle affectée par les blessures sélectionnées parmi l'eczéma pour le traiter.

16. Matériau pour la guérison des blessures pour l'utilisation selon la revendication 1, dans lequel le matériau est un textile comprenant des fibres incorporant l'oxyde de cuivre (I) et/ou l'oxyde de cuivre (II), pour la mise en contact avec une surface corporelle ayant des blessures sélectionnées parmi les plaies, les exulcérations et les brûlures pour leur traitement et leur guérison.

17. Matériau pour la guérison des blessures pour l'utilisation selon la revendication 1, dans lequel le matériau est un matériau de suture comprenant des fibres incorporant l'oxyde de cuivre (I) et/ou l'oxyde de cuivre (II).

18. Matériau pour la guérison des blessures pour l'utilisation selon la revendication 1, dans lequel le matériau est un matériau de suture comprenant un filament polymère ayant des particules microscopiques d'oxyde de cuivre (I) et/ou d'oxyde de cuivre (II) sous une forme pulvérulente enchâssées directement à l'intérieur avec une partie desdites particules qui est exposée et qui fait saillie de leurs surfaces.

19. Matériau pour la guérison des blessures pour l'utilisation selon la revendication 1, dans lequel le matériau est un textile de guérison des blessures à utiliser dans un uniforme militaire ou un article de vêtement de dessous ou de dessus, ledit textile incluant des fibres incorporant l'oxyde de cuivre (I) et/ou l'oxyde de cuivre (II) qui à l'issue de l'entrée dans une blessure d'un personnel militaire blessé qui le porte, permet d'obtenir à la fois un effet antibactérien et un effet de guérison sur ladite blessure.

20. Matériau pour la guérison des blessures pour l'utilisation selon la revendication 1, dans lequel le matériau est un uniforme militaire ou un article de vêtement de dessous ou de dessus comprenant des fibres incorporant l'oxyde de cuivre (I) et/ou l'oxyde de cuivre (II), lesquelles fibres lors de l'entrée dans une blessure d'un personnel militaire blessé qui le porte, permet d'obtenir à la fois un effet antibactérien et un effet de guérison sur ladite blessure.

21. Matériau pour la guérison des blessures pour l'utilisation selon la revendication 1, dans lequel le matériau est un textile de guérison des blessures ou un film, un filament ou une gaine extrudé(e) de guérison des blessures comprenant l'oxyde de cuivre (I) et/ou l'oxyde de cuivre (II), pour la mise en contact avec une surface corporelle présentant des blessures sélectionnées parmi les plaies, les exulcérations, les ulcérations, les lésions, les ouvertures cutanées ou les brûlures pour leur traitement et leur guérison.

22. Matériau pour la guérison des blessures pour l'utilisation selon la revendication 1, dans lequel le matériau est un vêtement choisi parmi le groupe constitué des pyjamas, des chemises de nuit et des sous-vêtements pour vêtement hospitalier et d'installations de soin de santé pour les patients, ledit vêtement ayant un panneau incluant l'oxyde de cuivre (I) et/ou l'oxyde de cuivre (II) incorporé au moins dans la zone du vêtement qui s'étend de manière adjacente aux fesses d'un patient, pour la prévention et la guérison des blessures sélectionnées parmi les plaies de lit et les plaies de pression.

23. Matériau pour la guérison des blessures pour l'utilisation selon l'une quelconque des revendications 2, 5, 7, 8, 10-12, 14, 15 et 20, dans lequel lesdites fibres sont des fibres polymères ayant lesdites particules microscopiques d'oxyde de cuivre sous une forme pulvérulente enchâssées directement à l'intérieur avec une portion desdites particules qui est exposée et qui fait saillie de leurs surfaces.

24. Matériau pour la guérison des blessures pour l'utilisation selon l'une quelconque des revendications 2, 5, 7, 8, 10-12, 14, 15 et 20, dans lequel lesdites fibres sont des fibres cellulosiques plaquées d'oxyde de cuivre.
